(19) Europäisches Patentamt
European Patent Office
Office européen des brevets

(11) Veröffentlichungsnummer : **0 342 459 B1**

(12) # EUROPÄISCHE PATENTSCHRIFT

(45) Veröffentlichungstag der Patentschrift : **20.03.91 Patentblatt 91/12**

(21) Anmeldenummer : **89108219.0**

(22) Anmeldetag : **06.05.89**

(51) Int. Cl.[5] : **C07C 69/65,** A01N 37/10, C07C 69/734, A01N 37/36, C07C 323/56, C07C 69/736, C07C 69/616

(54) **Acrylester und diese enthaltende Fungizide.**

(30) Priorität : **14.05.88 DE 3816577**

(43) Veröffentlichungstag der Anmeldung : **23.11.89 Patentblatt 89/47**

(45) Bekanntmachung des Hinweises auf die Patenterteilung : **20.03.91 Patentblatt 91/12**

(84) Benannte Vertragsstaaten : **AT BE CH DE ES FR GB GR IT LI NL SE**

(56) Entgegenhaltungen :
**EP-A- 0 178 826**
**EP-A- 0 203 606**
**EP-A- 0 260 832**
**CHEMICAL ABSTRACTS OF JAPAN, Band 95, 1981, Seite 639, Zusammenfassung Nr. 168044c, Columbus, Ohio, US; M.F. EL-NEWAIHY et al.: "Condensation of 3,4-disubstituted acetophenones with dimethyl homophthalate"**
**THE JOURNAL OF ORGANIC CHEMISTRY, Band 53, Nr. 3, 5. Februar 1988, American Chemical Society, Columbus, Ohio, US; T. TSUDA et al.: "Palladium(0)-catalyzed coupling reaction of lithium (alfa-carbalkoxyvinyl)-cuprates with organic halides"**

(73) Patentinhaber : **BASF Aktiengesellschaft**
**Carl-Bosch-Strasse 38**
**W-6700 Ludwigshafen (DE)**

(72) Erfinder : **Brand, Siegbert, Dr.**
**Hegelstrasse 39**
**W-6940 Weinheim (DE)**
Erfinder : **Schütz, Franz, Dr.**
**Budapester Strasse 45**
**W-6700 Ludwigshafen (DE)**
Erfinder : **Wenderoth, Bernd, Dr.**
**Schwalbenstrasse 26**
**W-6840 Lampertheim (DE)**
Erfinder : **Sauter, Hubert, Dr.**
**Neckarpromenade 20**
**W-6800 Mannheim 1 (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

EP 0 342 459 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Acrylester, diese enthaltende Fungizide und ihre Verwendung als Fungizide.

Es ist bekannt, das N-Tridecyl-2,6-dimethylmorpholin oder seine Salze, z.B. das Acetat, als Fungizide zu verwenden (DE-1 164 152, DE-1 173 722). Es ist ferner bekannt, α-phenyl-β-methoxyacrylsäuremethylester-derivate (DE-3 519 282.8) als Fungizide zu verwenden.

Ihre Wirkung ist jedoch in manchen Fällen ungenügend.

Es wurde nun gefunden, daß neue Acrylsäureester der Formel I

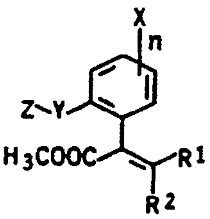

(I)

in der

$R^1$ und $R^2$ gleich oder verschieden sind und Halogen, $C_{1-4}$-Alkyl, $C_{1-3}$-Alkoxy, $C_{1-3}$-Alkylthio, Oxiranyl gegebenenfalls substituiert durch Halogen, Hydroxy, $C_{1-3}$-Alkoxy, $C_{1-3}$-Halogenalkyl oder $C_{1-3}$-Halogenalkoxy bedeuten oder $R^1$ und $R^2$ zusammen einen gegebenenfalls ungesättigten 3 bis 6-gliedrigen Ring bedeuten, der gegebenenfalls 1 bis 3 Heteroatome O oder $S(O)_m$ (m = 0,1 oder 2) enthält und gegebenenfalls durch Halogen, Hydroxy, $C_{1-3}$-Alkyl, $C_{1-3}$-Alkoxy, $C_{1-3}$-Halogenalkyl oder Oxo substituiert ist, und

X gleich oder verschieden ist und Wasserstoff, Halogen, Cyano, Nitro, gegebenenfalls substituiertes $C_1$-$C_4$-Alkyl, gegebenenfalls substituiertes $C_1$-$C_4$-Alkoxy, gegebenenfalls substituiertes Halogen-$C_1$-$C_4$-alkyl,

n 1 bis 4,

Y gegebenenfalls substituiertes $C_1$-$C_4$-Alkylen, $C_2$-$C_4$-Alkenylen, $C_2$-$C_4$-Alkinylen, O, $S(O)_p$ (p = 0,1 oder 2), gegebenenfalls $C_1$-$C_4$-alkyl-substituiertes N, Oxycarbonyl, Carbonyloxy, Carbonyloxy-$C_1$-$C_4$-alkylen, Oxycarbonyl-$C_1$-$C_4$-alkylen, Oxy-$C_1$-$C_4$-alkylen, Thio-$C_1$-$C_4$-alkylen, Azo, Carbonylamino, Aminocarbonyl,

$>NCOO-$,

Methylenoxy, Methylenthio und

Z Wasserstoff, Alkyl, Cycloalkyl, Aryl, Arylalkyl, Aralkenyl, Aralkoxy, Aryloxy, Aryloxyalkyl, Hetaryl, Alkoxy, Alkoxyalkyl, Alkylthio, Halogenalkyl bedeutet, wobei diese Reste gegebenenfalls substituiert sind durch Halogen, Cyano, Nitro, Alkyl, Alkenyl, Halogenalkenyl, Alkoxy, Halogenalkyl, gegebenenfalls substituiertes Phenyl, Alkoxycarbonyl, bedeuten, eine sehr gute fungizide Wirkung haben.

Die neuen Verbindungen der Formel I können bei der Herstellung als Gemische von Stereoisomeren (E, Z-Isomere, Diastereomere, Enantiomere) anfallen, die in üblicher Weise, z.B. durch Kristallisation oder Chromatographie, in die Einzelkomponenten getrennt werden können. Sowohl die einzelnen Isomeren als auch ihre Gemische können als Fungizide verwendet werden und werden von der Erfindung erfaßt.

$R^1$, $R^2$ sind gleich oder verschieden und sind bevorzugt Methyl, Ethyl, n- und i-Propyl, Fluor, Chlor, Brom,

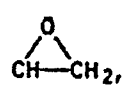

$OCH_3$, $OC_2H_5$, $CH_2OCH_3$, $SCH_3$, $S-C_2H_5$, $CF_3$ oder $R^1$ und $R^2$ zusammen sind bevorzugt $-(CH_2)-_q$ (q = 3, 4 oder 5), $-CH=CH-CH=CH-$, $-O(CH_2)_2O-$, $-O(CH_2)_3O-$, $-OCH_2C(CH_3)_2CH_2O-$, $-S(CH_2)_2S-$, $-S(CH_2)_3S-$,

$$-\overset{O}{\overset{\|}{C}}O(CH_2)_2-,$$

$-CH_2O(CH_2)_2-$.

n ist bevorzugt null.

Y ist bevorzugt Methylen, Ethylen, Ethenylen, Ethinylen, O,S, Methylenoxy, Oxymethylen, Carbonyloxy, Oxycarbonyl,

$$>NCO-O-,$$

Z ist bevorzugt Wasserstoff, $C_1$-$C_{16}$-Alkyl, $C_2$-$C_{16}$-Alkenyl, $C_2$-$C_{16}$-Alkinyl, (z.B. Methyl, Ethyl, n- und isopropyl, n-Butyl-, tert. Butyl-, 2,2-Dimethylpropyl, 2-Ethyl-hex-1-yl, 2,6-Dimethyl-hept-1-yl, 2,6,10-Trimethylundec-1-yl, Allyl, Prenyl, Geranyl, 2,3-Dihydrogeranyl, Tetrahydrogeranyl, 2,6-Dimethyl-hepta-1,5-dien-1-yl, 2,6-Dimethylhept-5-en-1-yl, 2-Ethylhex-1-en-1-yl), die gegebenenfalls mit $C_1$-$C_4$-Alkoxy, Halogen substituiert sein können, oder Z weiterhin bevorzugt mit $C_1$-$C_4$-Alkyl oder Halogen oder $C_1$-$C_4$-Alkoxy substituiertes $C_3$-$C_{12}$-Cycloalkyl (z.B. Cyclohexyl, Cyclopropyl, insbesondere 1-Methylcyclopropyl, 2-Methylcyclopropyl, 2,2-Dichlorcyclopropyl oder

2,2-Dichlor-1-Methyl-cyclopropyl (A1)
2,2-Dichlor-3,3-Dimethyl-cyclopropyl (A2)
2,2,3,3-Tetramethylcyclopropyl (A3)
2-(2'-Methyl-1'-propenyl-)3,3-Dimethylcyclopropyl (A4)
2-(2',2'-Difluorvinyl-)3,3-Dimethylcyclopropyl (A5)
2-(2',2'-Dichlorvinyl)-3,3-Dimethylcyclopropyl (A6)
2-(2',2'-Dibromvinyl)-3,3-Dimethylcyclopropyl (A7)
2-Phenylcyclopropyl (A8)
2-(4-Chlorphenyl)cyclopropyl (A9)
2,2-Dichlor-3-Phenylcyclopropyl (A10)
2-Carbomethoxy-cyclopropyl (A11)

weiterhin bevorzugt Aryl, insbesondere Phenyl, Phenoxyphenyl, Phenyl-phenyl, $C_1$-$C_4$-Alkoxyphenyl, Trifluormethylphenyl, Halogenphenyl, $C_1$-$C_4$-Alkylphenyl, (z.B. 2-Fluor-, 3-Fluor-, 4-Fluor-, 2-Chlor-, 3-Chlor-, 4-Chlor-, 2-Chlor, 6-Fluor-, 2,4-Dichlor-, 2,6-Dichlor-, 3,5-Dichlor-, 2,4,6-Trichlor-, 2-Brom, 4-Brom, 2-Methyl-, 3-Methyl-, 4-Methyl-, 2,4-Dimethyl-, 2,6-Dimethyl-, 2,4,6-Trimethyl-, 4-tert. Butyl-, 2-Methoxy-, 3-Methoxy-, 4-Methoxy-, 2,3-Dimethoxy-, 2-$CF_3$–, 3-$CF_3$–, 4-$CF_3$–, 3-tert. Butoxy, 4-tert. Butoxy-, 4-phenoxy-, 4-phenylphenyl und Naphthyl (1- und 2-Naphthyl),

Aryloxy (z.B. phenoxy)

Aralkyl, phenyl-$C_1$-$C_4$-Alkyl (z.B. Benzyl, Halogenbenzyl, $C_1$-$C_4$-Alkylbenzyl, 2-Chlorbenzyl, 2-Methylbenzyl, 2-phenylethyl, 3-Phenylpropyl, 4-Phenylbutyl)

Aralkenyl (z.B. Styryl, Halogenstyryl, $C_1$-$C_4$-Alkylstyryl, ortho-Chlor-Styryl, para-tert.Butyl-Styryl)

Hetaryl mit 5 oder 6 Ringgliedern und Stickstoff, Sauerstoff oder Schwefel als Heteroatom (z.B. 2-pyridyl-, 2-pyrimidyl, 2-Furyl-, 2-Thiophenyl-)

Aryloxy-$C_1$-$C_4$-alkyl (z.B. phenoxyethyl, 4-phenoxybutyl),

Halogen-$C_1$-$C_4$-alkyl (z.B. Trifluormethyl, Trichlormethyl)

Halogen-$C_1$-$C_4$-alkoxy (z.B. Trifluormethoxy, Trichlormethoxy)

Die Verbindungen der Formel I können z.B. hergestellt werden durch die in Schema 1 gezeigten Umsetzungen.

Schema 1:

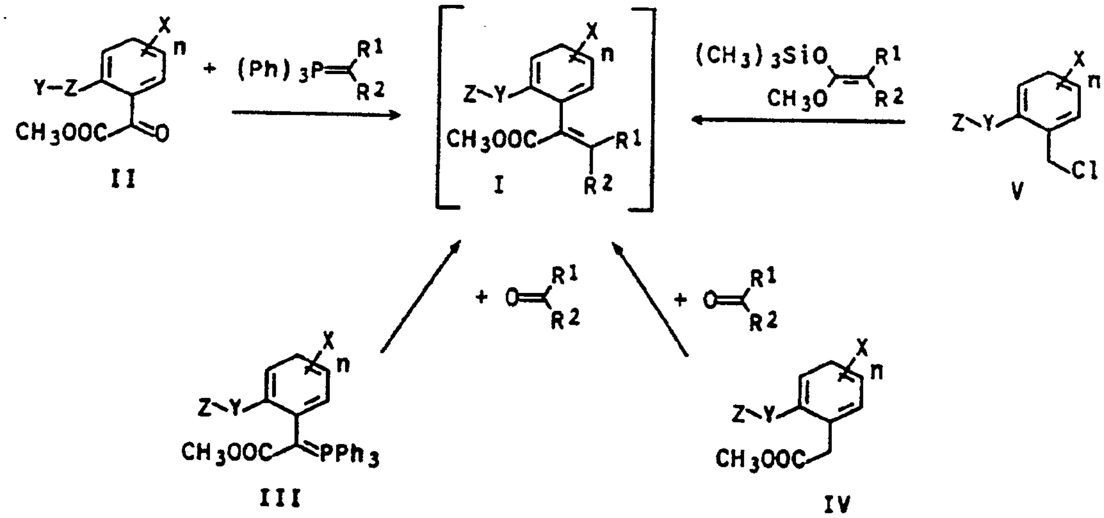

II I V III IV

Die α-Ketocarbonsäureester der Formel II lassen sich über eine Wittig-Reaktion mit einem disubstituierten Phosphoniumhalogenid in an sich bekannter Weise in Gegenwart einer Base wie z.B. n-Butyllithium, Kalium-tert.-butylat, Natriumhydrid oder Natriummethylat in die Acrylester der Formel I umwandeln (vgl. R. Raap, Can. J. Chem. 49, 2143 (1971)).

Ylide der Formel III reagieren mit Ketonen ebenfalls zu produkten der Formel I (vgl. G. Wittig, U. Schöllkopf, Org. Synth. Coll. Vol. V 751 (1973)).

Die Umsetzung eines Phenylchlorcarbens V mit einem Silylenolester in Gegenwart eines Radikalfängers liefert in an sich bekannter Weise (N. Slougui, G. Rousseau, Synth. Commun. 12 (5) 401 (1982)) β-disubstituierte Acrylester der Formel I.

Die Esterderivate der Formel I erhält man auch durch Perkin-Kondensation einer phenylessigsäure mit Ketonen in Anwesenheit von Acetanhydrid und einer Base (vgl. Johnson Org. Reactions 1, 210 (1942)) mit anschließender Veresterung.

Die Herstellung der α-Ketoester der Formel II ist bekannt (Ep 178826). So setzt man z.B. die aromatischen Grignardverbindungen VI mit Imidazoliden der Formel VII um (J.S. Nimitz, H.S. Mosher, J. Org. Chem. 46, 211 (1981)), wobei die Reste $x_n$, y, Z, R die oben genannten Bedeutungen haben.

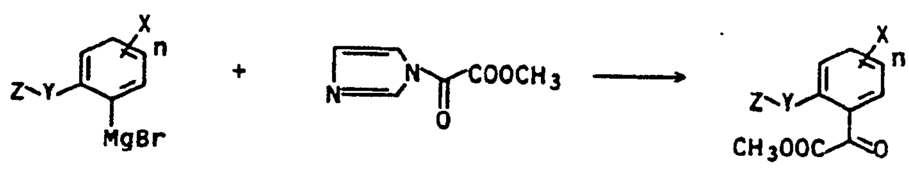

VI VII II

Die Derivate der Formel II in denen Y Oxymethylen, Thiomethylen oder Carboxymethylen und n = 0 bedeutet stellt man vorteilhaft auf die in Schema 2 gezeigte Weise her.

Schema 2

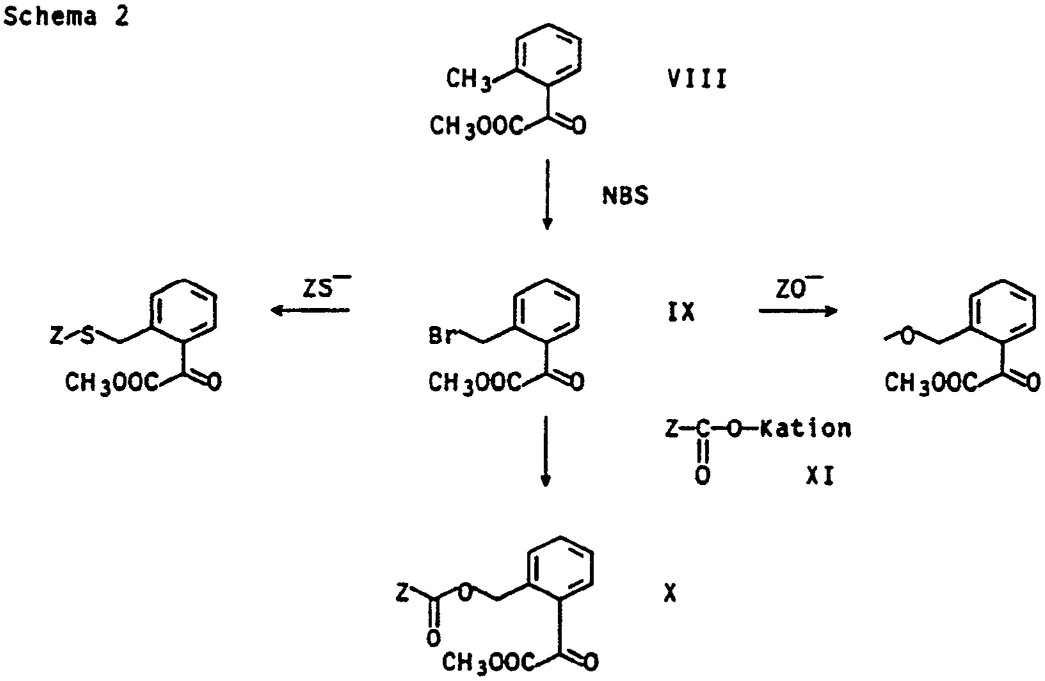


α-Ketoester der Formel VIII lassen sich mit Brom oder N-Bromsuccinimid (NBS) in z.B. Tetrachlormethan, ggf. unter Belichtung mit einer Lichtquelle geeigneter Wellenlänge (z.B. Hg-Dampf-Lampe, 300 Watt) (vgl. Horner, Winkelmann, Angew. Chem. 71, 349 (1959)) in die Benzylbromide der Formel IX überführen.

Die neuen α-Ketocarbonsäureester der Formel IX sind wertvolle Zwischenprodukte. Sie können z.B. zu den Derivaten der Formel X umgesetzt werden, indem man sie mit einem Alkali-, Erdalkali- oder Ammoniumsalz einer Carbonsäure der Formel XI, worin Z die oben angegebene Bedeutung hat, in einem Lösungs- oder Verdünnungsmittel, z.B. Aceton, Acetonitril, Dimethylsulfoxid, Dioxan, Dimethylformamid, N-Methylpyrrolidon, N-Methylpyrrolidon, N,N'-Dimethylpropylenharnstoff, Pyridin und gegebenenfalls unter Zusatz eines Katalysators, z.B. 0,01 – 10 Gew.% – bezogen auf Verbindung X–Tetramethylethylendiamin oder Kaliumiodid zur Reaktion bringt.

Die Acrylesterderivate der allgemeinen Formel XII – lassen sich durch die in Schema 3 gezeigten Umsetzungen herstellen.

Schema 3:

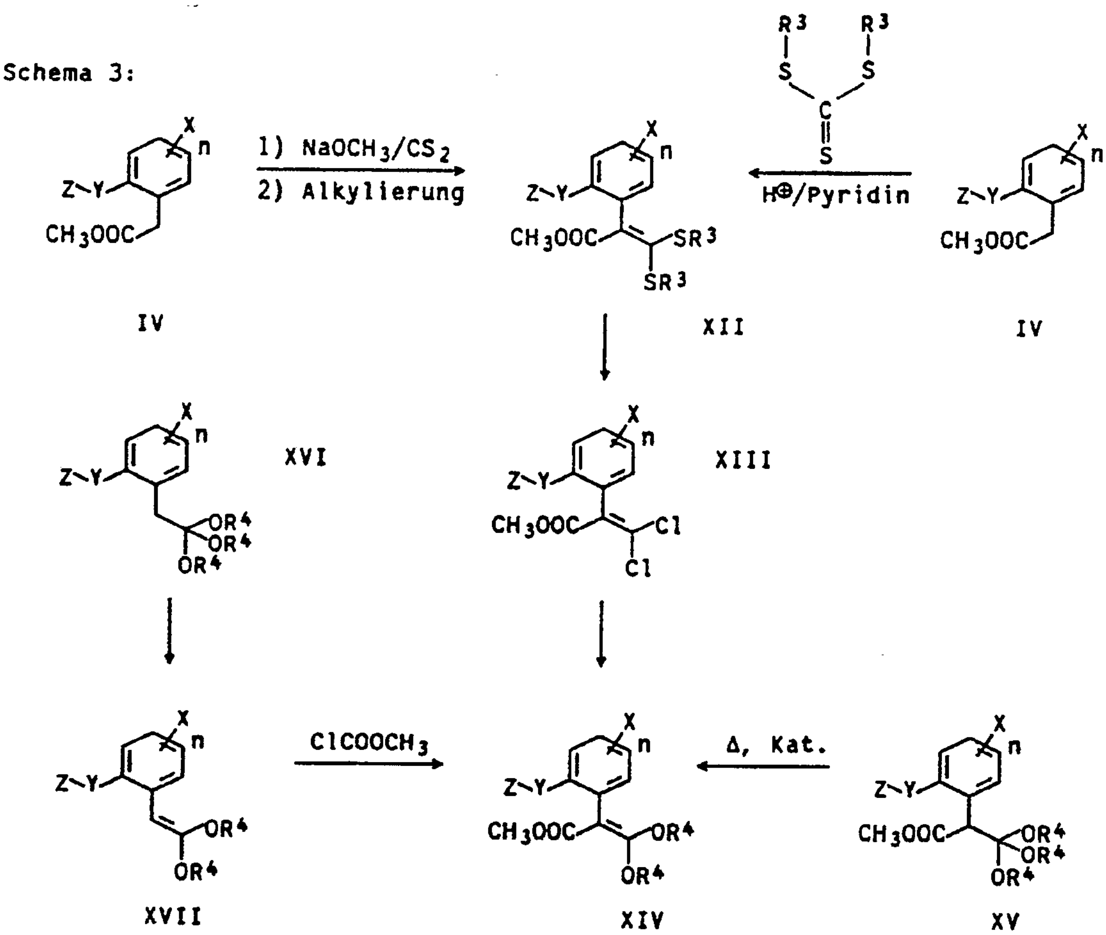


IV, XII, IV, XVI, XIII, XVII, XIV, XV

($R^3$, $R^4$ ist Alkyl oder Alkylen ; s. Tab. 3)

Ketenmercaptale der Formel XII lassen sich durch Alkylierung der aus Phenylessigestern (IV) und Schwefelkohlenstoff erhältlichen Salze von Dithiocarbonsäuren (vgl. R. Gompper, W. Töpfl Chem. Ber. 95 (1962) 2861 und J. L. Ann. Chem. 659 (1962), 90) bzw. durch Reaktion von Trithiocarbonaten mit aktiven Methylenkomponenten (vgl. R. Gompper, E. Kutter Chem. Ber. 98, 1365 (1965)) herstellen.

Ihre Umwandlung zu den β,β-Dichloracrylestern der Formel XIII erfolgt mit Phosphorpentachlorid (vgl. R. Gompper etal. Angew. Chem. 76 (1964) 583).

Die Umsetzung dieser aktivierten Dichlorethylene zu den Ketenacetalen der Formel XIV erreicht man durch Umsetzung mit Alkoholat (vgl. S.M.Mc Elvain, H.F. Mc Shane J. Am. Chem. Soc. 74 (1952) 2662).

Ein weiterer Zugang zu den β,β-Dialkoxyacrylaten der Formel XIV besteht in der basenkatalysierten Alkohol-Eliminierung aus den Ortho-carbonsäure-triestern der Formel XV bzw. XVI. (vgl. S.M. Mc Elvain etal. J. Am. Chem. Soc. 73, 206 (1951), ibid, 71, 47 (1949)), wobei im Falle von Arylketenacetalen (XVII) eine Acylierung mit Chlorameisensäuremethylester folgt (vgl. S.M. Mc Elvain, R.D. Mullineaux J. Am. Chem. Soc. 74, 1811 (1952)).

Die Herstellung der neuen Verbindungen der Formel I wird durch folgende Beispiele erläutert :

1. Herstellung von 3-Methyl-2-[2'-(α-Methylcyclopropylcarboxymethylen)-phenyl]-2-butensäuremethylester (Nr. 280, Tab. 1)

a) Herstellung von ortho-Brommethylphenylglyoxylsäuremethylester 50,0 g (0,28 mol) ortho-Methyl-Phenylglyoxylsäuremethylester werden zusammen mit 50,0 g (0,28 mol) frisch kristallisiertem N-Bromsuccinimid in 3 l Tetrachlorkohlenstoff gelöst. Man belichtet eine Stunde mit einer 300 W-Hg-Dampf-Lampe, engt die Reaktionsmischung auf ca. 1 l ein und wäscht die organische phase mit 3 × 200 ml Wasser. Nach dem Trocknen über Natriumsulfat und abdestillieren des Lösemittels wird der Rückstand an Kieselgel mit Methyl-tert. Butylether/Hexan 1 : 9 chromatographiert. Man erhält 10 g Ketoester (II, yZ = $CH_3$) und 21,1 g Benzylbromid (II, YZ = $CH_2Br$) (37% bezogen auf umgesetzten Ketoester) als gelbes öl.

$^1H$ ($CDCl_3$) : δ = 3,97(s, 3H), 4,90(s, 2H), 7,4-7,8(m, 4H)
IR (Film) : 2955 ; 1740, 1690 ; 1435, 1318, 1207, 999 $cm^{-1}$

b) Herstellung von ortho-(α-Methylcyclopropylcarboxymethylen)-phenylglyoxylsäuremethylester
13,8 g (0,1 mol) des Kaliumsalzes der α-Methylcyclopropancarbonsäure werden mit 21,1 g (0,082 mol) ortho-(Brommethyl)-phenylglyoxylsäure und 0,3 g Kaliumiodid in 300 ml N-Methylpyrrolidon gelöst. Man läßt 15 h bei 23°C rühren, gießt auf 300 ml Eiswasser und extrahiert mit 3 × 200 ml Methyl-tert. Butylether. Die organischen Phasen werden mit Wasser gewaschen, mit Natriumsulfat getrocknet und eingeengt. Man erhält 24,0 g (106%) chromatographisch reinen Ester, der in die Folgereaktion eingesetzt wird.

$^1H$($CDCl_3$) : δ = 0,70(m, 2H), 1,27(m, 2H), 1,35(s, 3H), 3,96(s, 3H), 5,46(s, 2H), 7,4-7,8(m, 4H).

c) Herstellung von 3-Methyl-2[2'-(α-Methylcyclopropylcarboxymethylen)-phenyl]-2-butensäure-methylester (Nr. 280, Tab. 1)
13,8 g (0,032 mol) iso-Propyltriphenylphosphoniumiodid werden unter Stickstoff in 100 ml wasserfreiem Tetrahydrofuran vorgelegt. Bei 0°C tropft man 20 ml einer 1,6 molaren Lösung von n-Butyllithium in Hexan zu. Nach 1 h bei 0°C werden 8,0 g (0,029 mol) in 40 ml wasserfreiem Tetrahydrofuran gelöster ortho-(α-Methyl-cyclopropylcarboxymethylen)-phenylglyoxysäuremethyl-ester zugetropft. Man läßt auf 23°C erwärmen, rührt weitere 2 h, gießt auf gesättigte Ammoniumchloridlösung und extrahiert mit Methyl-tert. butylether. Die organischen Phasen werden mit Wasser gewaschen, getrocknet (Natriumsulfat) und eingeengt. Das Rohprodukt wird durch Säulenchroamtographie an Kieselgel mit Methyltert.-Butylether/Hexan 1 : 9 aufgetrennt. Man erhält 1,9 g (22%) des obengenannten Esters als öl.

$^1H$($CDCl_3$) : δ = 0,65(m, 2H), 1,25(m, 2H), 1,30(s, 3H), 1,60(s, 3H), 2,25(s, 3H), 3,65(s, 3H), 5,00(s, 2H), 7,0-7,5(m, 4H)

IR (Film) : 1720, 1630, 1322, 1222, 1156, 1090, 755 $cm^{-1}$

2. Herstellung von 3-Methyl-2-[2'(2"-Methylbenzyloxy)phenyl]-2-butensäuremethylester (Nr. 73, Tab. 1)
8,64 g (20 mmol) iso-Propylphosphoniumiodid werden bei 0°C in 50 ml wasserfreiem Tetrahydrofuran unter Stickstoff vorgelegt. 8,3 ml 2,5 M n-Butyllithiumlösung in Hexan werden bei 0°C zugetropft. Nach 30 min bei dieser Temperatur gibt man 5,8 g (20 mmol) 2-(2'-Methylbenzyloxy)-phenylglyoxylsäuremethylester in 15 ml wasserfreiem Tetrahydrofuran in einem Schuß zu. Es wird 15 h bei Raumtemperatur (23°C) gerührt, am Rotationsverdampfer eingeengt und der Rückstand zwischen gesättigter Ammoniumchloridlösung und Methyl-tert.-butylether/Hexan 1 : 1 verteilt. Dreimaliges Extrahieren der wässrigen phase mit diesem Lösungsmittelgemisch, Trocknen der organischen phasen über Natriumsulfat und Einengen führt zu einem Rohprodukt, das durch Säulenchromatographie an Kieselgel mit Methyl-tert.-Butylether/Hexan 1 : 2 aufgetrennt wird. Man erhält 3,3 g (53%) des obengenannten Esters als hellgelbes öl.

$^1H$-NMR ($CDCl_3$) : δ = 1,67(s, 3H), 2,22(s, 3H), 2,40(2, 3H), 3,57(s, 3H), 5,05(s, 3H), 6,9-7,4 (m, 8H)

IR(Film) : 1714, 1600, 1490, 1449, 1302, 1270, 1224, 1088, 752 $cm^{-1}$
In entsprechender Weise können die in den folgenden Tabellen aufgeführten Verbindungen hergestellt werden.

Tabelle 1

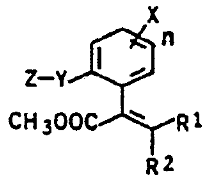

(X = Wasserstoff)

| Nr. | $R^1$ | $R^2$ | Y | Z | Daten |
|---|---|---|---|---|---|
| 1 | $CH_3$ | $CH_3$ | $CH_2CH_2$ | $C_6H_5$ | |
| 2 | $CH_3$ | $CH_3$ | $CH_2CH_2$ | 2-F-$C_6H_4$ | |
| 3 | $CH_3$ | $CH_3$ | $CH_2CH_2$ | 3-F-$C_6H_4$ | |
| 4 | $CH_3$ | $CH_3$ | $CH_2CH_2$ | 4-F-$C_6H_4$ | |
| 5 | $CH_3$ | $CH_3$ | $CH_2CH_2$ | 2-Cl-$C_6H_4$ | |
| 6 | $CH_3$ | $CH_3$ | $CH_2CH_2$ | 3-Cl-$C_6H_4$ | |
| 7 | $CH_3$ | $CH_3$ | $CH_2CH_2$ | 4-Cl-$C_6H_4$ | |
| 8 | $CH_3$ | $CH_3$ | $CH_2CH_2$ | 2Cl,6F-$C_6H_3$ | |
| 9 | $CH_3$ | $CH_3$ | $CH_2CH_2$ | 2,4-$Cl_2$-$C_6H_3$ | |
| 10 | $CH_3$ | $CH_3$ | $CH_2CH_2$ | 2,6-$Cl_2$-$C_6H_3$ | |
| 11 | $CH_3$ | $CH_3$ | $CH_2CH_2$ | 3,5-$Cl_2$-$C_6H_3$ | |
| 12 | $CH_3$ | $CH_3$ | $CH_2CH_2$ | 2,4,6-$Cl_3$-$C_6H_2$ | |
| 13 | $CH_3$ | $CH_3$ | $CH_2CH_2$ | 2-$CH_3$-$C_6H_4$ | |
| 14 | $CH_3$ | $CH_3$ | $CH_2CH_2$ | 3-$CH_3$-$C_6H_4$ | |
| 15 | $CH_3$ | $CH_3$ | $CH_2CH_2$ | 4-$CH_3$-$C_6H_4$ | |
| 16 | $CH_3$ | $CH_3$ | $CH_2CH_2$ | 4-t-$C_4H_9$-$C_6H_4$ | |
| 17 | $CH_3$ | $CH_3$ | $CH_2CH_2$ | 2,4-$(CH_3)_2$-$C_6H_3$ | |
| 18 | $CH_3$ | $CH_3$ | $CH_2CH_2$ | 2,6-$(CH_3)_2$-$C_6H_3$ | |
| 19 | $CH_3$ | $CH_3$ | $CH_2CH_2$ | 2,4,6-$(CH_3)_3$-$C_6H_2$ | |
| 20 | $CH_3$ | $CH_3$ | $CH_2CH_2$ | 2-$OCH_3$-$C_6H_4$ | |
| 21 | $CH_3$ | $CH_3$ | $CH_2CH_2$ | 3-$OCH_3$-$C_6H_4$ | |
| 22 | $CH_3$ | $CH_3$ | $CH_2CH_2$ | 4-$OCH_3$-$C_6H_4$ | |
| 23 | $CH_3$ | $CH_3$ | $CH_2CH_2$ | 2-$CF_3$-$C_6H_4$ | |
| 24 | $CH_3$ | $CH_3$ | $CH_2CH_2$ | 3-$CF_3$-$C_6H_4$ | |
| 25 | $CH_3$ | $CH_3$ | $CH_2CH_2$ | 4-$CF_3$-$C_6H_4$ | |
| 26 | $CH_3$ | $CH_3$ | $CH_2CH_2$ | 4-O-t-$C_4H_9$-$C_6H_4$ | |
| 27 | $CH_3$ | $CH_3$ | $CH_2CH_2$ | 4-O-$C_6H_5$-$C_6H_4$ | |
| 28 | $CH_3$ | $CH_3$ | $CH_2CH_2$ | 4-$C_6H_5$-$C_6H_4$ | |
| 29 | $CH_3$ | $CH_3$ | $CH_2CH_2$ | 2-Br-$C_6H_4$ | |
| 30 | $CH_3$ | $CH_3$ | $CH_2CH_2$ | 4-Br-$C_6H_4$ | |
| 31 | $CH_3$ | $CH_3$ | -CH=CH- | $C_6H_5$ | |
| 32 | $CH_3$ | $CH_3$ | -CH=CH- | 2-F-$C_6H_4$ | |
| 33 | $CH_3$ | $CH_3$ | -CH=CH- | 3-F-$C_6H_4$ | |
| 34 | $CH_3$ | $CH_3$ | -CH=CH- | 4-F-$C_6H_4$ | |
| 35 | $CH_3$ | $CH_3$ | -CH=CH- | 2-Cl-$C_6H_4$ | |

| Nr. | $R^1$ | $R^2$ | Y | Z | Daten |
|---|---|---|---|---|---|
| 36 | $CH_3$ | $CH_3$ | -CH=CH- | $3-Cl-C_6H_4$ | |
| 37 | $CH_3$ | $CH_3$ | -CH=CH- | $4-Cl-C_6H_4$ | |
| 38 | $CH_3$ | $CH_3$ | -CH=CH- | $2Cl,6F-C_6H_3$ | |
| 39 | $CH_3$ | $CH_3$ | -CH=CH- | $2,4-Cl_2-C_6H_3$ | |
| 40 | $CH_3$ | $CH_3$ | -CH=CH- | $2,6-Cl_2-C_6H_3$ | |
| 41 | $CH_3$ | $CH_3$ | -CH=CH- | $3,5-Cl_2-C_6H_3$ | |
| 42 | $CH_3$ | $CH_3$ | -CH=CH- | $2,4,6-Cl_3-C_6H_2$ | |
| 43 | $CH_3$ | $CH_3$ | -CH=CH- | $2-CH_3-C_6H_4$ | |
| 44 | $CH_3$ | $CH_3$ | -CH=CH- | $3-CH_3-C_6H_4$ | |
| 45 | $CH_3$ | $CH_3$ | -CH=CH- | $4-CH_3-C_6H_4$ | |
| 46 | $CH_3$ | $CH_3$ | -CH=CH- | $4-t-C_4H_9-C_6H_4$ | |
| 47 | $CH_3$ | $CH_3$ | -CH=CH- | $2,4-(CH_3)_2-C_6H_3$ | |
| 48 | $CH_3$ | $CH_3$ | -CH=CH- | $2,6-(CH_3)_2-C_6H_3$ | |
| 49 | $CH_3$ | $CH_3$ | -CH=CH- | $2,4,6-(CH_3)_3-C_6H_2$ | |
| 50 | $CH_3$ | $CH_3$ | -CH=CH- | $2-OCH_3-C_6H_4$ | |
| 51 | $CH_3$ | $CH_3$ | -CH=CH- | $3-OCH_3-C_6H_4$ | |
| 52 | $CH_3$ | $CH_3$ | -CH=CH- | $4-OCH_3-C_6H_4$ | |
| 53 | $CH_3$ | $CH_3$ | -CH=CH- | $2-CF_3-C_6H_4$ | |
| 54 | $CH_3$ | $CH_3$ | -CH=CH- | $3-CF_3-C_6H_4$ | |
| 55 | $CH_3$ | $CH_3$ | -CH=CH- | $4-CF_3-C_6H_4$ | |
| 56 | $CH_3$ | $CH_3$ | -CH=CH- | $4-O-t-C_4H_9-C_6H_4$ | |
| 57 | $CH_3$ | $CH_3$ | -CH=CH- | $4-O-C_6H_5-C_6H_4$ | |
| 58 | $CH_3$ | $CH_3$ | -CH=CH- | $4-C_6H_5-C_6H_4$ | |
| 59 | $CH_3$ | $CH_3$ | -CH=CH- | $2-Br-C_6H_4$ | |
| 60 | $CH_3$ | $CH_3$ | -CH=CH- | $4-Br-C_6H_4$ | |
| 61 | $CH_3$ | $CH_3$ | $-CH_2O-$ | $C_6H_5$ | |
| 62 | $CH_3$ | $CH_3$ | $-CH_2O-$ | $2-F-C_6H_4$ | |
| 63 | $CH_3$ | $CH_3$ | $-CH_2O-$ | $3-F-C_6H_4$ | |
| 64 | $CH_3$ | $CH_3$ | $-CH_2O-$ | $4-F-C_6H_4$ | |
| 65 | $CH_3$ | $CH_3$ | $-CH_2O-$ | $2-Cl-C_6H_4$ | |
| 66 | $CH_3$ | $CH_3$ | $-CH_2O-$ | $3-Cl-C_6H_4$ | |
| 67 | $CH_3$ | $CH_3$ | $-CH_2O-$ | $4-Cl-C_6H_4$ | |
| 68 | $CH_3$ | $CH_3$ | $-CH_2O-$ | $2Cl,6F-C_6H_3$ | |
| 69 | $CH_3$ | $CH_3$ | $-CH_2O-$ | $2,4-Cl_2-C_6H_3$ | |
| 70 | $CH_3$ | $CH_3$ | $-CH_2O-$ | $2,6-Cl_2-C_6H_3$ | |
| 71 | $CH_3$ | $CH_3$ | $-CH_2O-$ | $3,5-Cl_2-C_6H_3$ | |
| 72 | $CH_3$ | $CH_3$ | $-CH_2O-$ | $2,4,6-Cl_3-C_6H_2$ | |
| 73 | $CH_3$ | $CH_3$ | $-CH_2O-$ | $2-CH_3-C_6H_4$ | öl; IR(Film): 1713, 1630, 1276, 1215, 1112, 1092, 758, 708 |
| 74 | $CH_3$ | $CH_3$ | $-CH_2O-$ | $3-CH_3-C_6H_4$ | |
| 75 | $CH_3$ | $CH_3$ | $-CH_2O-$ | $4-CH_3-C_6H_4$ | |

| Nr. | $R^1$ | $R^2$ | Y | Z | Daten |
|---|---|---|---|---|---|
| 76 | $CH_3$ | $CH_3$ | $-CH_2O-$ | $4-t-C_4H_9-C_6H_4$ | |
| 77 | $CH_3$ | $CH_3$ | $-CH_2O-$ | $2,4-(CH_3)_2-C_6H_3$ | |
| 78 | $CH_3$ | $CH_3$ | $-CH_2O-$ | $2,6-(CH_3)_2-C_6H_3$ | |
| 79 | $CH_3$ | $CH_3$ | $-CH_2O-$ | $2,4,6-(CH_3)_3-C_6H_2$ | |
| 80 | $CH_3$ | $CH_3$ | $-CH_2O-$ | $2-OCH_3-C_6H_4$ | |
| 81 | $CH_3$ | $CH_3$ | $-CH_2O-$ | $3-OCH_3-C_6H_4$ | |
| 82 | $CH_3$ | $CH_3$ | $-CH_2O-$ | $4-OCH_3-C_6H_4$ | |
| 83 | $CH_3$ | $CH_3$ | $-CH_2O-$ | $2-CF_3-C_6H_4$ | |
| 84 | $CH_3$ | $CH_3$ | $-CH_2O-$ | $3-CF_3-C_6H_4$ | |
| 85 | $CH_3$ | $CH_3$ | $-CH_2O-$ | $4-CF_3-C_6H_4$ | |
| 86 | $CH_3$ | $CH_3$ | $-CH_2O-$ | $4-O-t-C_4H_9-C_6H_4$ | |
| 87 | $CH_3$ | $CH_3$ | $-CH_2O-$ | $4-O-C_6H_5-C_6H_4$ | |
| 88 | $CH_3$ | $CH_3$ | $-CH_2O-$ | $4-C_6H_5-C_6H_4$ | |
| 89 | $CH_3$ | $CH_3$ | $-CH_2O-$ | $2-Br-C_6H_4$ | |
| 90 | $CH_3$ | $CH_3$ | $-CH_2O-$ | $4-Br-C_6H_4$ | |
| 91 | $CH_3$ | $CH_3$ | $-OCH_2-$ | $C_6H_5$ | |
| 92 | $CH_3$ | $CH_3$ | $-OCH_2-$ | $2-F-C_6H_4$ | |
| 93 | $CH_3$ | $CH_3$ | $-OCH_2-$ | $3-F-C_6H_4$ | |
| 94 | $CH_3$ | $CH_3$ | $-OCH_2-$ | $4-F-C_6H_4$ | |
| 95 | $CH_3$ | $CH_3$ | $-OCH_2-$ | $2-Cl-C_6H_4$ | Oel; IR(Film): 1713, 1587, 1482, 1295, 1234, 1062, 749 |
| 96 | $CH_3$ | $CH_3$ | $-OCH_2-$ | $3-Cl-C_6H_4$ | |
| 97 | $CH_3$ | $CH_3$ | $-OCH_2-$ | $4-Cl-C_6H_4$ | |
| 98 | $CH_3$ | $CH_3$ | $-OCH_2-$ | $2Cl,6F-C_6H_3$ | |
| 99 | $CH_3$ | $CH_3$ | $-OCH_2-$ | $2,4-Cl_2-C_6H_3$ | |
| 100 | $CH_3$ | $CH_3$ | $-OCH_2-$ | $2,6-Cl_2-C_6H_3$ | |
| 101 | $CH_3$ | $CH_3$ | $-OCH_2-$ | $3,5-Cl_2-C_6H_3$ | |
| 102 | $CH_3$ | $CH_3$ | $-OCH_2-$ | $2,4,6-Cl_3-C_6H_2$ | |
| 103 | $CH_3$ | $CH_3$ | $-OCH_2-$ | $2-CH_3-C_6H_4$ | |
| 104 | $CH_3$ | $CH_3$ | $-OCH_2-$ | $3-CH_3-C_6H_4$ | |
| 105 | $CH_3$ | $CH_3$ | $-OCH_2-$ | $4-CH_3-C_6H_4$ | |
| 106 | $CH_3$ | $CH_3$ | $-OCH_2-$ | $4-t-C_4H_9-C_6H_4$ | |
| 107 | $CH_3$ | $CH_3$ | $-OCH_2-$ | $2,4-(CH_3)_2-C_6H_3$ | |
| 108 | $CH_3$ | $CH_3$ | $-OCH_2-$ | $2,6-(CH_3)_2-C_6H_3$ | |
| 109 | $CH_3$ | $CH_3$ | $-OCH_2-$ | $2,4,6-(CH_3)_3-C_6H_2$ | |
| 110 | $CH_3$ | $CH_3$ | $-OCH_2-$ | $2-OCH_3-C_6H_4$ | |
| 111 | $CH_3$ | $CH_3$ | $-OCH_2-$ | $3-OCH_3-C_6H_4$ | |
| 112 | $CH_3$ | $CH_3$ | $-OCH_2-$ | $4-OCH_3-C_6H_4$ | |
| 113 | $CH_3$ | $CH_3$ | $-OCH_2-$ | $2-CF_3-C_6H_4$ | |
| 114 | $CH_3$ | $CH_3$ | $-OCH_2-$ | $3-CF_3-C_6H_4$ | |
| 115 | $CH_3$ | $CH_3$ | $-OCH_2-$ | $4-CF_3-C_6H_4$ | |
| 116 | $CH_3$ | $CH_3$ | $-OCH_2-$ | $4-O-t-C_4H_9-C_6H_4$ | |
| 117 | $CH_3$ | $CH_3$ | $-OCH_2-$ | $4-O-C_6H_5-C_6H_4$ | |

| Nr. | $R^1$ | $R^2$ | Y | Z | Daten |
|---|---|---|---|---|---|
| 118 | $CH_3$ | $CH_3$ | $-OCH_2-$ | $4-C_6H_5-C_6H_4$ | |
| 119 | $CH_3$ | $CH_3$ | $-OCH_2-$ | $2-Br-C_6H_4$ | |
| 120 | $CH_3$ | $CH_3$ | $-OCH_2-$ | $4-Br-C_6H_4$ | |
| 121 | $CH_3$ | $CH_3$ | $-SCH_2-$ | $C_6H_5$ | |
| 122 | $CH_3$ | $CH_3$ | $-SCH_2-$ | $2-F-C_6H_4$ | |
| 123 | $CH_3$ | $CH_3$ | $-SCH_2-$ | $3-F-C_6H_4$ | |
| 124 | $CH_3$ | $CH_3$ | $-SCH_2-$ | $4-F-C_6H_4$ | |
| 125 | $CH_3$ | $CH_3$ | $-SCH_2-$ | $2-Cl-C_6H_4$ | |
| 126 | $CH_3$ | $CH_3$ | $-SCH_2-$ | $3-Cl-C_6H_4$ | |
| 127 | $CH_3$ | $CH_3$ | $-SCH_2-$ | $4-Cl-C_6H_4$ | |
| 128 | $CH_3$ | $CH_3$ | $-SCH_2-$ | $2Cl,6F-C_6H_3$ | |
| 129 | $CH_3$ | $CH_3$ | $-SCH_2-$ | $2,4-Cl_2-C_6H_3$ | |
| 130 | $CH_3$ | $CH_3$ | $-SCH_2-$ | $2,6-Cl_2-C_6H_3$ | |
| 131 | $CH_3$ | $CH_3$ | $-SCH_2-$ | $3,5-Cl_2-C_6H_3$ | |
| 132 | $CH_3$ | $CH_3$ | $-SCH_2-$ | $2,4,6-Cl_3-C_6H_2$ | |
| 133 | $CH_3$ | $CH_3$ | $-SCH_2-$ | $2-CH_3-C_6H_4$ | |
| 134 | $CH_3$ | $CH_3$ | $-SCH_2-$ | $3-CH_3-C_6H_4$ | |
| 135 | $CH_3$ | $CH_3$ | $-SCH_2-$ | $4-CH_3-C_6H_4$ | |
| 136 | $CH_3$ | $CH_3$ | $-SCH_2-$ | $4-t-C_4H_9-C_6H_4$ | |
| 137 | $CH_3$ | $CH_3$ | $-SCH_2-$ | $2,4-(CH_3)_2-C_6H_3$ | |
| 138 | $CH_3$ | $CH_3$ | $-SCH_2-$ | $2,6-(CH_3)_2-C_6H_3$ | |
| 139 | $CH_3$ | $CH_3$ | $-SCH_2-$ | $2,4,6-(CH_3)_3-C_6H_2$ | |
| 140 | $CH_3$ | $CH_3$ | $-SCH_2-$ | $2-OCH_3-C_6H_4$ | |
| 141 | $CH_3$ | $CH_3$ | $-SCH_2-$ | $3-OCH_3-C_6H_4$ | |
| 142 | $CH_3$ | $CH_3$ | $-SCH_2-$ | $4-OCH_3-C_6H_4$ | |
| 143 | $CH_3$ | $CH_3$ | $-SCH_2-$ | $2-CF_3-C_6H_4$ | |
| 144 | $CH_3$ | $CH_3$ | $-SCH_2-$ | $3-CF_3-C_6H_4$ | |
| 145 | $CH_3$ | $CH_3$ | $-SCH_2-$ | $4-CF_3-C_6H_4$ | |
| 146 | $CH_3$ | $CH_3$ | $-SCH_2-$ | $4-O-t-C_4H_9-C_6H_4$ | |
| 147 | $CH_3$ | $CH_3$ | $-SCH_2-$ | $4-O-C_6H_5-C_6H_4$ | |
| 148 | $CH_3$ | $CH_3$ | $-SCH_2-$ | $4-C_6H_5-C_6H_4$ | |
| 149 | $CH_3$ | $CH_3$ | $-SCH_2-$ | $2-Br-C_6H_4$ | |
| 150 | $CH_3$ | $CH_3$ | $-SCH_2-$ | $4-Br-C_6H_4$ | |
| 151 | $CH_3$ | $CH_3$ | $-CO-OCH_2-$ | $C_6H_5$ | Fp.: 75°C; IR(KBr): 1713, 1630, 1276, 1215, 1112, 1092, 758, 708 $cm^{-1}$ |
| 152 | $CH_3$ | $CH_3$ | $-CO-OCH_2-$ | $2-F-C_6H_4$ | |
| 153 | $CH_3$ | $CH_3$ | $-CO-OCH_2-$ | $3-F-C_6H_4$ | |
| 154 | $CH_3$ | $CH_3$ | $-CO-OCH_2-$ | $4-F-C_6H_4$ | |
| 155 | $CH_3$ | $CH_3$ | $-CO-OCH_2-$ | $2-Cl-C_6H_4$ | |
| 156 | $CH_3$ | $CH_3$ | $-CO-OCH_2-$ | $3-Cl-C_6H_4$ | |
| 157 | $CH_3$ | $CH_3$ | $-CO-OCH_2-$ | $4-Cl-C_6H_4$ | |

| Nr. | R1 | R2 | Y | Z | Daten |
|---|---|---|---|---|---|
| 158 | $CH_3$ | $CH_3$ | $-CO-OCH_2-$ | $2Cl,6F-C_6H_3$ | |
| 159 | $CH_3$ | $CH_3$ | $-CO-OCH_2-$ | $2,4-Cl_2-C_6H_3$ | |
| 160 | $CH_3$ | $CH_3$ | $-CO-OCH_2-$ | $2,6-Cl_2-C_6H_3$ | |
| 161 | $CH_3$ | $CH_3$ | $-CO-OCH_2-$ | $3,5-Cl_2-C_6H_3$ | |
| 162 | $CH_3$ | $CH_3$ | $-CO-OCH_2-$ | $2,4,6-Cl_3-C_6H_2$ | |
| 163 | $CH_3$ | $CH_3$ | $-CO-OCH_2-$ | $2-CH_3-C_6H_4$ | |
| 164 | $CH_3$ | $CH_3$ | $-CO-OCH_2-$ | $3-CH_3-C_6H_4$ | |
| 165 | $CH_3$ | $CH_3$ | $-CO-OCH_2-$ | $4-CH_3-C_6H_4$ | |
| 166 | $CH_3$ | $CH_3$ | $-CO-OCH_2-$ | $4-t-C_4H_9-C_6H_4$ | |
| 167 | $CH_3$ | $CH_3$ | $-CO-OCH_2-$ | $2,4-(CH_3)_2-C_6H_3$ | |
| 168 | $CH_3$ | $CH_3$ | $-CO-OCH_2-$ | $2,6-(CH_3)_2-C_6H_3$ | |
| 169 | $CH_3$ | $CH_3$ | $-CO-OCH_2-$ | $2,4,6-(CH_3)_3-C_6H_2$ | |
| 170 | $CH_3$ | $CH_3$ | $-CO-OCH_2-$ | $2-OCH_3-C_6H_4$ | |
| 171 | $CH_3$ | $CH_3$ | $-CO-OCH_2-$ | $3-OCH_3-C_6H_4$ | |
| 172 | $CH_3$ | $CH_3$ | $-CO-OCH_2-$ | $4-OCH_3-C_6H_4$ | |
| 173 | $CH_3$ | $CH_3$ | $-CO-OCH_2-$ | $2-CF_3-C_6H_4$ | |
| 174 | $CH_3$ | $CH_3$ | $-CO-OCH_2-$ | $3-CF_3-C_6H_4$ | |
| 175 | $CH_3$ | $CH_3$ | $-CO-OCH_2-$ | $4-CF_3-C_6H_4$ | |
| 176 | $CH_3$ | $CH_3$ | $-CO-OCH_2-$ | $4-O-t-C_4H_9-C_6H_4$ | |
| 177 | $CH_3$ | $CH_3$ | $-CO-OCH_2-$ | $4-O-C_6H_5-C_6H_4$ | |
| 178 | $CH_3$ | $CH_3$ | $-CO-OCH_2-$ | $4-C_6H_5-C_6H_4$ | |
| 179 | $CH_3$ | $CH_3$ | $-CO-OCH_2-$ | $2-Br-C_6H_4$ | |
| 180 | $CH_3$ | $CH_3$ | $-CO-OCH_2-$ | $4-Br-C_6H_4$ | |
| 181 | $CH_3$ | $CH_3$ | $-C\equiv C-$ | $C_6H_5$ | |
| 182 | $CH_3$ | $CH_3$ | $-C\equiv C-$ | $2-Cl-C_6H_4$ | |
| 183 | $CH_3$ | $CH_3$ | $-C\equiv C-$ | $3-Cl-C_6H_4$ | |
| 184 | $CH_3$ | $CH_3$ | $-C\equiv C-$ | $4-Cl-C_6H_4$ | |
| 185 | $CH_3$ | $CH_3$ | -O- | $C_6H_5$ | |
| 186 | $CH_3$ | $CH_3$ | -O- | $2-Cl-C_6H_4$ | |
| 187 | $CH_3$ | $CH_3$ | -O- | $3-Cl-C_6H_4$ | |
| 188 | $CH_3$ | $CH_3$ | -O- | $4-Cl-C_6H_4$ | |
| 189 | $CH_3$ | $CH_3$ | -O- | $2-CH_3-C_6H_4$ | |
| 190 | $CH_3$ | $CH_3$ | -O- | $4-CH_3-C_6H_4$ | |
| 191 | $CH_3$ | $CH_3$ | -O- | $4-C_6H_5-C_6H_4$ | |
| 192 | $CH_3$ | $CH_3$ | -O- | $1-C_{10}H_7$ | |
| 193 | $CH_3$ | $CH_3$ | -O- | $CH_3$ | |
| 194 | $CH_3$ | $CH_3$ | -O- | $CH_2CH=CH_2$ | |
| 195 | $CH_3$ | $CH_3$ | -O- | $CH_2CH=C(CH_3)_2$ | |
| 196 | $CH_3$ | $CH_3$ | -O- | Geranyl | |
| 197 | $CH_3$ | $CH_3$ | -O- | Tetrahydrogeranyl | |
| 198 | $CH_3$ | $CH_3$ | -O- | $2-OCH_3-C_6H_4$ | |
| 199 | $CH_3$ | $CH_3$ | -O- | $2,3-(OCH_3)_2-C_6H_3$ | |

| Nr. | $R^1$ | $R^2$ | Y | Z | Daten |
|---|---|---|---|---|---|
| 200 | $CH_3$ | $CH_3$ | -S- | $C_6H_5$ | |
| 201 | $CH_3$ | $CH_3$ | -S- | $2-Cl-C_6H_4$ | |
| 202 | $CH_3$ | $CH_3$ | -S- | $3-Cl-C_6H_4$ | |
| 203 | $CH_3$ | $CH_3$ | -S- | $4-Cl-C_6H_4$ | |
| 204 | $CH_3$ | $CH_3$ | -S- | $2-CH_3-C_6H_4$ | |
| 205 | $CH_3$ | $CH_3$ | -S- | $4-CH_3-C_6H_4$ | |
| 206 | $CH_3$ | $CH_3$ | -S- | $4-C_6H_5-C_6H_4$ | |
| 207 | $CH_3$ | $CH_3$ | -S- | $CH_3$ | |
| 208 | $CH_3$ | $CH_3$ | -S- | Geranyl | |
| 209 | $CH_3$ | $CH_3$ | -S- | 2-Pyridyl | |
| 210 | $CH_3$ | $CH_3$ | -S- | 2-Pyrimidyl | |
| 211 | $CH_3$ | $CH_3$ | -COO- | $C_6H_5$ | |
| 212 | $CH_3$ | $CH_3$ | -COO- | $2-Cl-C_6H_4$ | |
| 213 | $CH_3$ | $CH_3$ | -COO- | $3-Cl-C_6H_4$ | |
| 214 | $CH_3$ | $CH_3$ | -COO- | $4-Cl-C_6H_4$ | |
| 215 | $CH_3$ | $CH_3$ | -COO- | $2-CH_3-C_6H_4$ | |
| 216 | $CH_3$ | $CH_3$ | -COO- | $4-CH_3-C_6H_4$ | |
| 217 | $CH_3$ | $CH_3$ | -COO- | $4-C_6H_5-C_6H_4$ | |
| 218 | $CH_3$ | $CH_3$ | -O-CO- | $C_6H_5$ | |
| 219 | $CH_3$ | $CH_3$ | -O-CO- | $2-Cl-C_6H_4$ | |
| 220 | $CH_3$ | $CH_3$ | -O-CO- | $3-Cl-C_6H_4$ | |
| 221 | $CH_3$ | $CH_3$ | -O-CO- | $4-Cl-C_6H_4$ | |
| 222 | $CH_3$ | $CH_3$ | -O-CO- | $2-CH_3-C_6H_4$ | |
| 223 | $CH_3$ | $CH_3$ | -O-CO- | $CH_3$ | |
| 224 | $CH_3$ | $CH_3$ | -O-CO- | $t-C_4H_9$ | |
| 225 | $CH_3$ | $CH_3$ | -O-CO- | $CH_2C_6H_5$ | |
| 226 | $CH_3$ | $CH_3$ | -HNCO-O | $C_6H_5$ | |
| 227 | $CH_3$ | $CH_3$ | -HNCO-O | $2-Cl-C_6H_4$ | |
| 228 | $CH_3$ | $CH_3$ | -HNCO-O | $4-Cl-C_6H_4$ | |
| 229 | $CH_3$ | $CH_3$ | -HNCO-O | $C_6H_{11}$ | |
| 230 | $CH_3$ | $CH_3$ | -HNCO-O | $n-C_4H_9$ | |
| 231 | $CH_3$ | $CH_3$ | -CH=CH- | $CH_2C(CH_3)_3$ | |
| 232 | $CH_3$ | $CH_3$ | -CH=CH- | $CH=C(CH_3)CH_2CH_2CH=C(CH_3)_2$ | |
| 233 | $CH_3$ | $CH_3$ | -CH=CH- | $CH_2CH(CH_3)CH_2CH_2CH=C(CH_3)_2$ | |
| 234 | $CH_3$ | $CH_3$ | -CH=CH- | $CH=C(C_2H_5)CH_2CH_2CH_2CH_3$ | |
| 235 | $CH_3$ | $CH_3$ | -CH=CH- | $CH_2CH(C_2H_5)CH_2CH_2CH_2CH_3$ | |
| 236 | $CH_3$ | $CH_3$ | -CH=CH- | $CH_2CH(CH_3)CH_2CH_2CH_2CH(CH_3)_2$ | |
| 237 | $CH_3$ | $CH_3$ | -CH=CH- | Cyclopropyl | |
| 238 | $CH_3$ | $CH_3$ | -CH=CH- | 1-Methylcyclopropyl | |
| 239 | $CH_3$ | $CH_3$ | -CH=CH- | $CH=CH-C_6H_5$ | |
| 240 | $CH_3$ | $CH_3$ | -CH=CH- | $CH_2-CH_2-C_6H_5$ | |
| 241 | $CH_3$ | $CH_3$ | -CH=CH- | $C(CH_3)_3$ | |

| Nr. | $R^1$ | $R^2$ | Y | Z | Daten |
|---|---|---|---|---|---|
| 242 | $CH_3$ | $CH_3$ | $-CH=CH-$ | Cyclohexyl | |
| 243 | $CH_3$ | $CH_3$ | $-CH=CH-$ | $(CH_2CH(CH_3)CH_2CH_2)_2CH_2CH(CH_3)_2$ | |
| 244 | $CH_3$ | $CH_3$ | $-CH=CH-$ | 2-Furyl | |
| 245 | $CH_3$ | $CH_3$ | $-CH=CH-$ | 2-Thiophenyl | |
| 246 | $CH_3$ | $CH_3$ | $-CH=CH-$ | $CF_3$ | |
| 247 | $CH_3$ | $CH_3$ | $-CH=CH-$ | $CCl_3$ | |
| 248 | $CH_3$ | $CH_3$ | $-CH_2CH_2-$ | $C(CH_3)_3$ | |
| 249 | $CH_3$ | $CH_3$ | $-CH_2CH_2-$ | $CH_2C(CH_3)_3$ | |
| 250 | $CH_3$ | $CH_3$ | $-CH_2CH_2-$ | $CH_2CH(CH_3)CH_2CH_2CH_2CH(CH_3)_2$ | |
| 251 | $CH_3$ | $CH_3$ | $-CH_2CH_2-$ | $CH_2CH(C_2H_5)CH_2CH_2CH_2CH_3$ | |
| 252 | $CH_3$ | $CH_3$ | $-CH_2CH_2-$ | $(CH_2CH(CH_3)CH_2CH_2)_2CH_2CH(CH_3)_2$ | |
| 253 | $CH_3$ | $CH_3$ | $-CH_2CH_2-$ | Cyclopropyl | |
| 254 | $CH_3$ | $CH_3$ | $-CH_2CH_2-$ | 1-Methyl-cyclopropyl | |
| 255 | $CH_3$ | $CH_3$ | $-CH_2CH_2-$ | Cyclohexyl | |
| 256 | $CH_3$ | $CH_3$ | $-CH_2CH_2-$ | $CH_2CH_2C_6H_5$ | |
| 257 | $CH_3$ | $CH_3$ | $-CH_2CH_2-$ | $CH_2CH_2CH_2C_6H_5$ | |
| 258 | $CH_3$ | $CH_3$ | $-CH_2CH_2-$ | $CF_3$ | |
| 259 | $CH_3$ | $CH_3$ | $-CH_2CH_2-$ | $CCl_3$ | |
| 260 | $CH_3$ | $CH_3$ | $-CH_2CH_2-$ | $O-C_6H_5$ | |
| 261 | $CH_3$ | $CH_3$ | $-CH_2O-$ | $CH_2CH(CH_3)CH_2CH_2CH=C(CH_3)_2$ | |
| 262 | $CH_3$ | $CH_3$ | $-CH_2O-$ | $(CH=C(CH_3)CH_2CH_2)_2CH=C(CH_3)_2$ | |
| 263 | $CH_3$ | $CH_3$ | $-CH_2O-$ | $(CH_2CH(CH_3)CH_2CH_2)_2CH_2CH(CH_3)_2$ | |
| 264 | $CH_3$ | $CH_3$ | $-CH_2O-$ | $CH=CHC_6H_5$ | |
| 265 | $CH_3$ | $CH_3$ | $-CH_2O-$ | $CH_2-CH_2C_6H_5$ | |
| 266 | $CH_3$ | $CH_3$ | $-CH_2O-$ | $CH_2-O-C_6H_5$ | |
| 267 | $CH_3$ | $CH_3$ | $-OCH_2-$ | $CH_3$ | |
| 268 | $CH_3$ | $CH_3$ | $-OCH_2-$ | $C_2H_5$ | |
| 269 | $CH_3$ | $CH_3$ | $-OCH_2-$ | $n-C_3H_7$ | |
| 270 | $CH_3$ | $CH_3$ | $-OCH_2-$ | $i-C_3H_7$ | |
| 271 | $CH_3$ | $CH_3$ | $-OCH_2-$ | $CH_2CH=CH_2$ | |
| 272 | $CH_3$ | $CH_3$ | $-OCH_2-$ | $CH_2CH=C(CH_3)_2$ | |
| 273 | $CH_3$ | $CH_3$ | $-OCH_2-$ | $CH_2C_6H_5$ | |
| 274 | $CH_3$ | $CH_3$ | $-OCH_2-$ | $CH_2-(2-Cl-C_6H_4)$ | |
| 275 | $CH_3$ | $CH_3$ | $-OCH_2-$ | $CH_2(2-CH_3-C_6H_4)$ | |
| 276 | $CH_3$ | $CH_3$ | $-OCH_2-$ | $CH_2CH_2C_6H_5$ | |
| 277 | $CH_3$ | $CH_3$ | $-OCH_2-$ | $CH_2CH_2OC_6H_5$ | |
| 278 | $CH_3$ | $CH_3$ | $-OCH_2-$ | $(CH_2)_4OC_6H_5$ | |
| 279 | $CH_3$ | $CH_3$ | $-CO-OCH_2-$ | Cyclopropyl | öl; IR(Film): 1727, 1398, 1223, 1167, 1091, 755 |
| 280 | $CH_3$ | $CH_3$ | $-CO-OCH_2-$ | 1-Methylcyclopropyl | öl; IR(Film): 1720, 1322, 1222, 1156, 1090, 755 $cm^{-1}$ |

| Nr. | $R^1$ | $R^2$ | Y | Z | Daten |
|---|---|---|---|---|---|
| 281 | $CH_3$ | $CH_3$ | $-CO-OCH_2-$ | 2-Methylcyclopropyl | |
| 282 | $CH_3$ | $CH_3$ | $-CO-OCH_2-$ | 2,2-Dichlorcyclopropyl | |
| 283 | $CH_3$ | $CH_3$ | $-CO-OCH_2-$ | A1* | |
| 284 | $CH_3$ | $CH_3$ | $-CO-OCH_2-$ | A2* | |
| 285 | $CH_3$ | $CH_3$ | $-CO-OCH_2-$ | A3* | |
| 286 | $CH_3$ | $CH_3$ | $-CO-OCH_2-$ | A4* | |
| 287 | $CH_3$ | $CH_3$ | $-CO-OCH_2-$ | A5* | |
| 288 | $CH_3$ | $CH_3$ | $-CO-OCH_2-$ | A6* | |
| 289 | $CH_3$ | $CH_3$ | $-CO-OCH_2-$ | A7* | |
| 290 | $CH_3$ | $CH_3$ | $-CO-OCH_2-$ | A8* | |
| 291 | $CH_3$ | $CH_3$ | $-CO-OCH_2-$ | A9* | |
| 292 | $CH_3$ | $CH_3$ | $-CO-OCH_2-$ | A10* | |
| 293 | $CH_3$ | $CH_3$ | $-CO-OCH_2-$ | A11* | |
| 294 | $CH_3$ | $CH_3$ | $-CO-OCH_2-$ | $CH_3$ | |
| 295 | $CH_3$ | $CH_3$ | $-CO-OCH_2-$ | $C_2H_5$ | |
| 296 | $CH_3$ | $CH_3$ | $-CO-OCH_2-$ | $n-C_3H_7$ | |
| 297 | $CH_3$ | $CH_3$ | $-CO-OCH_2-$ | $i-C_3H_7$ | |
| 298 | $CH_3$ | $CH_3$ | $-CO-OCH_2-$ | $CH(CH_3)CH_2CH_3$ | |
| 299 | $CH_3$ | $CH_3$ | $-CO-OCH_2-$ | $tert-C_4H_9$ | Öl; IR(Film): 1727, 1718, 1282, 1222, 1150, 1090, 754 $cm^{-1}$ |
| 300 | $CH_3$ | $CH_3$ | $-CO-OCH_2-$ | $CH(CH_3)CH_2CH_2CH_2CH_3$ | |
| 301 | $CH_3$ | $CH_3$ | $-CO-OCH_2-$ | $CH(C_2H_5)CH_2CH_2CH_2CH_3$ | |
| 302 | $CH_3$ | $CH_3$ | $-CO-OCH_2-$ | $CH(iso-C_3H_7)CH_2CH_2CH(CH_3)_2$ | |
| 303 | $CH_3$ | $CH_3$ | $-CO-OCH_2-$ | $CH=CH-C_6H_5$ | |
| 304 | $CH_3$ | $CH_3$ | $-CO-OCH_2-$ | $CH=CH-(2-Chlor)-C_6H_4$ | |
| 305 | $CH_3$ | $CH_3$ | $-CO-OCH_2-$ | $CH=CH-(4-tert.Butyl)-C_6H_4$ | |
| 306 | $CH_3$ | $CH_3$ | $-CO-OCH_2-$ | $CH_2CH_2-C_6H_5$ | |
| 307 | $CH_3$ | $CH_3$ | $-CO-OCH_2-$ | $(CH_2)_4C_6H_5$ | |
| 308 | $CH_3$ | $CH_3$ | $-CO-OCH_2-$ | $CH_2CH_2-OC_6H_5$ | |
| 309 | $CH_3$ | $CH_3$ | $-CO-OCH_2-$ | $(CH_2)_4-OC_6H_5$ | |
| 310 | $CH_3$ | $CH_3$ | $-CO-OCH_2-$ | $CH(Cl)C_6H_5$ | |

* Formeln s. vorhergehender Text

Tabelle 2

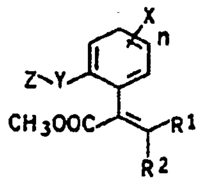

(X = Wasserstoff)

| Nr. | $R^1$ | $R^2$ | Y | Z | Daten |
|---|---|---|---|---|---|
| 1a | Cl | Cl | -CH=CH- | $C_6H_5$ | |
| 2a | Cl | Cl | $-CH_2CH_2$ | $C_6H_5$ | |
| 3a | Cl | Cl | $-OCH_2-$ | $C_6H_5$ | |
| 4a | Cl | Cl | $-CH_2-O-$ | $C_6H_5$ | |
| 5a | Cl | Cl | $-SCH_2-$ | $C_6H_5$ | |
| 6a | Cl | Cl | -O- | $C_6H_5$ | |
| 7a | Br | Br | -CH=CH- | $C_6H_5$ | |
| 8a | Br | Br | $-CH_2CH_2-$ | $C_6H_5$ | |
| 9a | Br | Br | $-OCH_2-$ | $C_6H_5$ | |
| 10a | Br | Br | $-CH_2O-$ | $C_6H_5$ | |
| 11a | Br | Br | $-SCH_2-$ | $C_6H_5$ | |
| 12a | Br | Br | -O- | $C_6H_5$ | |
| 13a | $C_2H_5$ | $C_2H_5$ | -CH=CH- | $C_6H_5$ | |
| 14a | $C_2H_5$ | $C_2H_5$ | $-CH_2CH_2-$ | $C_6H_5$ | |
| 15a | $C_2H_5$ | $C_2H_5$ | $-OCH_2-$ | $C_6H_5$ | |
| 16a | $C_2H_5$ | $C_2H_5$ | $-CH_2O-$ | $C_6H_5$ | |
| 17a | $C_2H_5$ | $C_2H_5$ | $-SCH_2-$ | $C_6H_5$ | |
| 18a | $C_2H_5$ | $C_2H_5$ | -O- | $C_6H_5$ | |
| 19a | $C_2H_5$ | $C_2H_5$ | $-OCH_2-$ | 2-Cl-$C_6H_4$ | |
| 20a | $C_2H_5$ | $C_2H_5$ | $-CH_2O-$ | 2-Cl-$C_6H_4$ | |
| 21a | $C_2H_5$ | $C_2H_5$ | -CO-$OCH_2$ | $C_6H_5$ | |
| 22a | $C_2H_5$ | $C_2H_5$ | -CO-$OCH_2$- | 1-Methylcyclopropyl | |
| 23a | $C_2H_5$ | $C_2H_5$ | -CO-$OCH_2$- | t-$C_4H_9$ | |
| 24a | $C_2H_5$ | $C_2H_5$ | -CO-$OCH_2$- | 2,2-Dichlorcyclopropyl | |
| 25a | $C_2H_5$ | $C_2H_5$ | -CO-$OCH_2$- | $CH(C_2H_5)CH_2CH_2CH_2CH_3$ | |
| 26a | $C_2H_5$ | $C_2H_5$ | -CO-$OCH_2$- | CH=CH-$C_6H_5$ | |
| 27a | $C_2H_5$ | $C_2H_5$ | -CO-$OCH_2$ | A1* | |
| 28a | $C_2H_5$ | $C_2H_5$ | -CO-$OCH_2$- | A6* | |
| 29a | n-$C_3H_7$ | n-$C_3H_7$ | -CH=CH- | $C_6H_5$ | |
| 30a | n-$C_3H_7$ | n-$C_3H_7$ | $-CH_2CH_2-$ | $C_6H_5$ | |
| 31a | n-$C_3H_7$ | n-$C_3H_7$ | $-CH^2O-$ | $c^6H^5$ | |
| 32a | n-$C_3H_7$ | n-$C_3H_7$ | $-OCH_2-$ | $C_6H_5$ | |
| 33a | n-$C_3H_7$ | n-$c^3H^7$ | -CO-$OCH^2$- | $c^6H^5$ | |
| 34a | $OCH_3$ | $OCH_3$ | -CH=CH- | $C_6H_5$ | |
| 35a | $OCH_3$ | $OCH_3$ | -CH=CH- | 3-Cl-$C_6H_4$ | |

| Nr. | $R^1$ | $R^2$ | Y | Z | Daten |
|---|---|---|---|---|---|
| 36a | $OCH_3$ | $OCH_3$ | -CH=CH- | $3\text{-}CF_3\text{-}C_6H_4$ | |
| 37a | $OCH_3$ | $OCH_3$ | -CH=CH- | $2\text{-}CH_3\text{-}C_6H_4$ | |
| 38a | $OCH_3$ | $OCH_3$ | -CH=CH- | $3\text{-}CH_3\text{-}C_6H_4$ | |
| 39a | $OCH_3$ | $OCH_3$ | $-CH_2CH_2-$ | $C_6H_5$ | |
| 40a | $OCH_3$ | $OCH_3$ | $-CH_2CH_2-$ | $3\text{-}Cl\text{-}C_6H_4$ | |
| 41a | $OCH_3$ | $OCH_3$ | $-CH_2CH_2-$ | $3\text{-}CF_3\text{-}C_6H_4$ | |
| 42a | $OCH_3$ | $OCH_3$ | $-CH_2CH_2-$ | $3\text{-}CH_3\text{-}C_6H_4$ | |
| 43a | $OCH_3$ | $OCH_3$ | $-CH_2CH_2-$ | $2\text{-}Cl\text{-}C_6H_4$ | |
| 44a | $OCH_3$ | $OCH_3$ | $-CH_2O-$ | $C_6H_5$ | |
| 45a | $OCH_3$ | $OCH_3$ | $-CH_2O-$ | $3\text{-}Cl\text{-}C_6H_4$ | |
| 46a | $OCH_3$ | $OCH_3$ | $-CH_2O-$ | $2,4\text{-}Cl_2\text{-}C_6H_3$ | |
| 47a | $OCH_3$ | $OCH_3$ | $-CH_2O-$ | $2\text{-}Br\text{-}C_6H_4$ | |
| 48a | $OCH_3$ | $OCH_3$ | $-OCH_2-$ | $C_6H_5$ | |
| 49a | $OCH_3$ | $OCH_3$ | $-OCH_2-$ | $3\text{-}Cl\text{-}C_6H_4$ | |
| 50a | $OCH_3$ | $OCH_3$ | $-OCH_2$ | $2,4\text{-}Cl_2\text{-}C_6H_3$ | |
| 51a | $OCH_3$ | $OCH_3$ | $-OCH_2-$ | $2\text{-}Br\text{-}C_6H_4$ | |
| 52a | $OCH_3$ | $OCH_3$ | $-S\text{-}CH_2-$ | $C_6H_5$ | |
| 53a | $OCH_3$ | $OCH_3$ | $-CO\text{-}OCH_2-$ | $C_6H_5$ | |
| 54a | $OCH_3$ | $OCH_3$ | $-CO\text{-}OCH_2-$ | tert.-$C_4H_9$ | |
| 55a | $OCH_3$ | $OCH_3$ | $-CO\text{-}OCH_2-$ | 1-Methylcyclopropyl | |
| 56a | $OCH_3$ | $OCH_3$ | $-CO\text{-}OCH_2-$ | 2,2-Dichlorcyclopropyl | |
| 57a | $OCH_3$ | $OCH_3$ | $-CO\text{-}OCH_2-$ | $CH(C_2H_5)CH_2CH_2CH_2CH_3$ | |
| 58a | $OCH_3$ | $OCH_3$ | $-CO\text{-}OCH_2-$ | $CH=CH\text{-}C_6H_5$ | |
| 59a | $OCH_3$ | $OCH_3$ | $-CO\text{-}OCH_2-$ | $C_6H_5$ | |
| 60a | $OCH_3$ | $OCH_3$ | $-CO\text{-}OCH_2-$ | A1* | |
| 61a | $OCH_3$ | $OCH_3$ | $-CO\text{-}OCH_2-$ | A6* | |
| 62a | $OC_2H_5$ | $OC_2H_5$ | -CH=CH- | $C_6H_5$ | |
| 63a | $OC_2H_5$ | $OC_2H_5$ | $-CH_2\text{-}CH_2-$ | $C_6H_5$ | |
| 64a | $OC_2H_5$ | $OC_2H_5$ | $-CH_2O-$ | $C_6H_5$ | |
| 65a | $OC_2H_5$ | $OC_2H_5$ | $-OCH_2-$ | $C_6H_5$ | |
| 66a | $OC_2H_5$ | $OC_2H_5$ | $-SCH_2-$ | $C_6H_5$ | |
| 67a | $OC_2H_5$ | $OC_2H_5$ | -O- | $C_6H_5$ | |
| 68a | $OC_2H_5$ | $OC_2H_5$ | $-CO\text{-}OCH_2-$ | $C_6H_5$ | |
| 69a | $OC_2H_5$ | $OC_2H_5$ | $-CO\text{-}OCH_2-$ | 1-Methylcyclopropyl | |
| 70a | $OC_2H_5$ | $OC_2H_5$ | $-CO\text{-}OCH_2-$ | A1* | |
| 71a | $OC_2H_5$ | $OC_2H_5$ | $-CO\text{-}OCH_2-$ | A6* | |
| 72a | $CH_2OCH_3$ | $CH_2OCH_3$ | -CH=CH- | $C_6H_5$ | |
| 73a | $CH_2OCH_3$ | $CH_2OCH_3$ | $-CH_2\text{-}CH_2-$ | $C_6H_5$ | |
| 74a | $CH_2OCH_3$ | $CH_2OCH_3$ | $-CH_2O-$ | $C_6H_5$ | |
| 75a | $CH_2OCH_3$ | $CH_2OCH_3$ | $-OCH_2-$ | $C_6H_5$ | |
| 76a | $CH_2OCH_3$ | $CH_2OCH_3$ | $-SCH_2-$ | $C_6H_5$ | |
| 77a | $CH_2OCH_3$ | $CH_2OCH_3$ | -O- | $C_6H_5$ | |

| Nr. | R1 | R2 | Y | Z | Daten |
|---|---|---|---|---|---|
| 78a | $CH_2OCH_3$ | $CH_2OCH_3$ | $-CO-OCH_2-$ | $C_6H_5$ | |
| 79a | $CH_2OCH_3$ | $CH_2OCH_3$ | $-CO-OCH_2-$ | 1-Methylcyclopropyl | |
| 80a | $SCH_3$ | $SCH_3$ | -CH=CH- | $C_6H_5$ | |
| 81a | $SCH_3$ | $SCH_3$ | -CH=CH- | $2-CH_3-C_6H_4$ | |
| 82a | $SCH_3$ | $SCH_3$ | $-CH_2CH_2-$ | $C_6H_5$ | |
| 83a | $SCH_3$ | $SCH_3$ | $-CH_2CH_2-$ | $3-Cl-C_6H_4$ | |
| 84a | $SCH_3$ | $SCH_3$ | $-CH_2CH_2-$ | $3-CH_3-C_6H_4$ | |
| 85a | $SCH_3$ | $SCH_3$ | $-CH_2CH_2-$ | $3-CF_3-C_6H_4$ | |
| 86a | $SCH_3$ | $SCH_3$ | $-CH_2O-$ | $C_6H_5$ | |
| 87a | $SCH_3$ | $SCH_3$ | $-CH_2O-$ | $3-Cl-C_6H_4$ | |
| 88a | $SCH_3$ | $SCH_3$ | $-OCH_2-$ | $C_6H_5$ | Öl; 1H-NMR ($CDCl_3$): $\delta$=2.10(3H),2.42(3H), 3,62(3H),5.10(2H), 6.9-7.1(2H),7.2-7.4(7H) |
| 89a | $SCH_3$ | $SCH_3$ | $-S-CH_2-$ | $C_6H_5$ | |
| 90a | $SCH_3$ | $SCH_3$ | $-CO-OCH_2-$ | $C_6H_5$ | |
| 91a | $SCH_3$ | $SCH_3$ | $-CO-OCH_2-$ | 1-Methylcyclopropyl | |
| 92a | $SCH_3$ | $SCH_3$ | $-CO-OCH_2-$ | A1* | |
| 93a | $SCH_3$ | $SCH_3$ | $-CO-OCH_2-$ | A6 | |
| 94a | $SC_2H_5$ | $SC_2H_5$ | -CH=CH- | $C_6H_5$ | |
| 95a | $SC_2H_5$ | $SC_2H_5$ | $-CH_2CH_2-$ | $C_6H_5$ | |
| 96a | $SC_2H_5$ | $SC_2H_5$ | $-CH_2O-$ | $C_6H_5$ | |
| 97a | $SC_2H_5$ | $SC_2H_5$ | $-OCH_2-$ | $C_6H_5$ | |
| 98a | $SC_2H_5$ | $SC_2H_5$ | $-SCH_2-$ | $C_6H_5$ | |
| 99a | $SC_2H_5$ | $SC_2H_5$ | -O- | $C_6H_5$ | |
| 100a | $SC_2H_5$ | $SC_2H_5$ | $-CO-OCH_2-$ | $C_6H_5$ | |
| 101a | $SC_2H_5$ | $SC_2H_5$ | $-CO-OCH_2$ | 1-Methylcyclopropyl | |
| 102a | $CF_3$ | $CF_3$ | -CH=CH- | $C_6H_5$ | |
| 103a | $CF_3$ | $CF_3$ | $-CH_2CH_2-$ | $C_6H_5$ | |
| 104a | $CF_3$ | $CF_3$ | $-CH_2O-$ | $C_6H_5$ | |
| 105a | $CF_3$ | $CF_3$ | $-OCH_2-$ | $C_6H_5$ | |
| 106a | $CF_3$ | $CF_3$ | $-SCH_2-$ | $C_6H_5$ | |
| 107a | $CF_3$ | $CF_3$ | -O- | $C_6H_5$ | |
| 108a | $CF_3$ | $CF_3$ | $-CO-OCH_2-$ | $C_6H_5$ | |
| 109a | $CF_3$ | $CF_3$ | $-CO-OCH_2-$ | 1-Methylcyclopropyl | |
| 110a | $CH_3$ | $C_2H_5$ | -CH=CH- | $C_6H_5$ | |
| 111a | $C_2H_5$ | $CH_3$ | -CH=CH- | $C_6H_5$ | |
| 112a | $CH_3$ | $C_2H_5$ | $-CH_2CH_2-$ | $C_6H_5$ | |
| 113a | $C_2H_5$ | $CH_3$ | $-CH_2CH_2-$ | $C_6H_5$ | |
| 114a | $CH_3$ | $C_2H_5$ | $-CH_2O-$ | $C_6H_5$ | |
| 115a | $C_2H_5$ | $CH_3$ | $-CH_2O-$ | $C_6H_5$ | |
| 116a | $CH_3$ | $C_2H_5$ | $-OCH_2-$ | $C_6H_5$ | |
| 117a | $C_2H_5$ | $CH_3$ | $-OCH_2-$ | $C_6H_5$ | |
| 118a | $CH_3$ | $C_2H_5$ | $-CO-OCH_2-$ | $C_6H_5$ | |
| 119a | $C_2H_5$ | $CH_3$ | $-CO-OCH_2-$ | $C_6H_5$ | |

| Nr. | $R^1$ | $R^2$ | Y | Z | Daten |
|---|---|---|---|---|---|
| 120a | $CH_3$ | $C_2H_5$ | $-CO-OCH_2-$ | 1-Methylcyclopropyl | |
| 121a | $C_2H_5$ | $CH_3$ | $-CO-OCH_2-$ | 1-Methylcyclopropyl | |
| 122a | $CH_3$ | $n-C_3H_7$ | $-CH=CH-$ | $C_6H_5$ | |
| 123a | $n-C_3H_7$ | $CH_3$ | $-CH=CH-$ | $C_6H_5$ | |
| 124a | $CH_3$ | $n-C_3H_7$ | $-CH_2CH_2-$ | $C_6H_5$ | |
| 125a | $n-C_3H_7$ | $CH_3$ | $-CH_2CH_2-$ | $C_6H_5$ | |
| 126a | $CH_3$ | $n-C_3H_7$ | $-CH_2O-$ | $C_6H_5$ | |
| 127a | $n-C_3H_7$ | $CH_3$ | $-CH_2O-$ | $C_6H_5$ | |
| 128a | $CH_3$ | $n-C_3H_7$ | $-OCH_2-$ | $C_6H_5$ | |
| 129a | $n-C_3H_7$ | $CH_3$ | $-OCH_2-$ | $C_6H_5$ | |
| 130a | $CH_3$ | $n-C_3H_7$ | $-CO-OCH_2-$ | $C_6H_5$ | |
| 131a | $n-C_3H_7$ | $CH_3$ | $-CO-OCH_2-$ | $C_6H_5$ | |
| 132a | $CH_3$ | $n-C_3H_7$ | $-CO-OCH_2-$ | 1-Methylcyclopropyl | |
| 133a | $n-C_3H_7$ | $CH_3$ | $-CO-OCH_2-$ | 1-Methylcyclopropyl | |
| 134a | $CH_3$ | $i-C_3H_7$ | $-CH=CH-$ | $C_6H_5$ | |
| 135a | $i-C_3H_7$ | $CH_3$ | $-CH=CH-$ | $C_6H_5$ | |
| 136a | $CH_3$ | $i-C_3H_7$ | $-CH_2CH_2-$ | $C_6H_5$ | |
| 137a | $i-C_3H_7$ | $CH_3$ | $-CH_2CH_2-$ | $C_6H_5$ | |
| 138a | $CH_3$ | $i-CH_3H_7$ | $-CH_2O-$ | $C_6H_5$ | |
| 139a | $i-C_3H_7$ | $CH_3$ | $-CH_2O-$ | $C_6H_5$ | |
| 140a | $CH_3$ | $i-C_3H_7$ | $-OCH_2-$ | $C_6H_5$ | |
| 141a | $i-C_3H_7$ | $CH_3$ | $-OCH_2-$ | $C_6H_5$ | |
| 142a | $CH_3$ | $i-C_3H_7$ | $-CO-OCH_2-$ | $C_6H_5$ | |
| 143a | $i-C_3H_7$ | $CH_3$ | $-CO-OCH_2-$ | $C_6H_5$ | |
| 144a | $CH_3$ | $i-C_3H_7$ | $-CO-OCH_2-$ | 1-Methylcyclopropyl | |
| 145a | $i-C_3H_7$ | $CH_3$ | $-CO-OCH_2-$ | 1-Methylcyclopropyl | |
| 146a | $CH_3$ | $OCH_3$ | $-CH=CH-$ | $C_6H_5$ | |
| 147a | $OCH_3$ | $CH_3$ | $-CH=CH-$ | $C_6H_5$ | |
| 148a | $CH_3$ | $OCH_3$ | $-CH_2CH_2-$ | $C_6H_5$ | |
| 149a | $OCH_3$ | $CH_3$ | $-CH_2CH_2-$ | $C_6H_5$ | |
| 150a | $CH_3$ | $OCH_3$ | $-CH_2O-$ | $C_6H_5$ | |
| 151a | $OCH_3$ | $CH_3$ | $-CH_2O-$ | $C_6H_5$ | |
| 152a | $CH_3$ | $OCH_3$ | $-OCH_2-$ | $C_6H_5$ | |
| 153a | $OCH_3$ | $CH_3$ | $-OCH_2-$ | $C_6H_5$ | |
| 154a | $CH_3$ | $OCH_3$ | $-CO-OCH_2-$ | $C_6H_5$ | |
| 155a | $OCH_3$ | $CH_3$ | $-CO-OCH_2-$ | $C_6H_5$ | |
| 156a | $CH_3$ | $OCH_3$ | $-CO-OCH_2-$ | 1-Methylcyclopropyl | |
| 157a | $OCH_3$ | $CH_3$ | $-CO-OCH_2-$ | 1-Methylcyclopropyl | |
| 158a | $C_2H_5$ | $OCH_3$ | $-CH=CH-$ | $C_6H_5$ | |
| 159a | $OCH_3$ | $C_2H_5$ | $-CH=CH-$ | $C_6H_5$ | |
| 160a | $C_2H_5$ | $OCH_3$ | $-CH_2CH_2-$ | $C_6H_5$ | |
| 161a | $OCH_3$ | $C_2H_5$ | $-CH_2CH_2-$ | $C_6H_5$ | |

| Nr. | $R^1$ | $R^2$ | Y | Z | Daten |
|---|---|---|---|---|---|
| 162a | $C_2H_5$ | $OCH_3$ | $-CH_2O-$ | $C_6H_5$ | |
| 163a | $OCH_3$ | $C_2H_5$ | $-CH_2O-$ | $C_6H_5$ | |
| 164a | $C_2H_5$ | $OCH_3$ | $-OCH_2$ | $C_6H_5$ | |
| 165a | $OCH_3$ | $C_2H_5$ | $-OCH_2-$ | $C_6H_5$ | |
| 166a | $C_2H_5$ | $OCH_3$ | $-CO-OCH_2-$ | $C_6H_5$ | |
| 167a | $OCH_3$ | $C_2H_5$ | $-CO-OCH_2-$ | $C_6H_5$ | |
| 168a | $C_2H_5$ | $OCH_3$ | $-CO-OCH_2-$ | 1-Methylcyclopropyl | |
| 169a | $OCH_3$ | $C_2H_5$ | $-CO-OCH_2-$ | 1-Methylcyclopropyl | |
| 170a | $CH_3$ | $SCH_3$ | $-CH=CH-$ | $C_6H_5$ | |
| 171a | $SCH_3$ | $CH_3$ | $-CH=CH-$ | $C_6H_5$ | |
| 172a | $CH_3$ | $SCH_3$ | $-CH_2CH_2-$ | $C_6H_5$ | |
| 173a | $SCH_3$ | $CH_3$ | $-CH_2CH_2-$ | $C_6H_5$ | |
| 174a | $CH_3$ | $SCH_3$ | $-CH_2O-$ | $C_6H_5$ | |
| 175a | $SCH_3$ | $CH_3$ | $-CH_2O-$ | $C_6H_5$ | |
| 176a | $CH_3$ | $SCH_3$ | $-OCH_2-$ | $C_6H_5$ | |
| 177a | $SCH_3$ | $CH_3$ | $-OCH_2-$ | $C_6H_5$ | |
| 178a | $CH_3$ | $SCH_3$ | $-CO-OCH_2-$ | $C_6H_5$ | |
| 179a | $SCH_3$ | $CH_3$ | $-CO-OCH_2-$ | $C_6H_5$ | |
| 180a | $CH_3$ | $SCH_3$ | $-CO-OCH_2-$ | 1-Methylcyclopropyl | |
| 181a | $SCH_3$ | $CH_3$ | $-CO-OCH_2$ | 1-Methylcyclopropyl | |
| 182a | $C_2H_5$ | $SCH_3$ | $-CH=CH-$ | $C_6H_5$ | |
| 183a | $SCH_3$ | $C_2H_5$ | $-CH=CH-$ | $C_6H_5$ | |
| 184a | $C_2H_5$ | $SCH_3$ | $-CH_2CH_2-$ | $C_6H_5$ | |
| 185a | $SCH_3$ | $C_2H_5$ | $-CH_2CH_2-$ | $C_6H_5$ | |
| 186a | $C_2H_5$ | $SCH_3$ | $-CH_2O-$ | $C_6H_5$ | |
| 187a | $SCH_3$ | $C_2H_5$ | $-CH_2O-$ | $C_6H_5$ | |
| 188a | $C_2H_5$ | $SCH_3$ | $-OCH_2-$ | $C_6H_5$ | |
| 189a | $SCH_3$ | $C_2H_5$ | $-OCH_3-$ | $C_6H_5$ | |
| 190a | $C_2H_5$ | $SCH_3$ | $-CO-OCH_2-$ | $C_6H_5$ | |
| 191a | $SCH_3$ | $C_2H_5$ | $-CO-OCH_2-$ | $C_6H_5$ | |
| 192a | $C_2H_5$ | $SCH_3$ | $-CO-OCH_2-$ | 1-Meth yclopropyl | |
| 193a | $SCH_3$ | $C_2H_5$ | $-CO-OCH_2-$ | 1-Meth, icyclopropyl | |
| 194a | $CH_3$ | 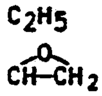 | $-CH=CH-$ | $C_6H_5$ | |
| 195a | 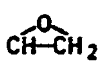 | $CH_3$ | $-CH=CH-$ | $C_6H_5$ | |
| 196a | $CH_3$ | 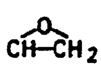 | $-CH_2CH_2-$ | $C_6H_5$ | |
| 197a | 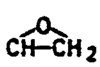 | $CH_3$ | $-CH_2CH_2-$ | $C_6H_5$ | |
| 198a | $CH_3$ | 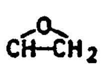 | $-CH_2O-$ | $C_6H_5$ | |

| Nr. | $R^1$ | $R^2$ | Y | Z | Daten |
|---|---|---|---|---|---|
| 199a | 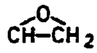 | $CH_3$ | $-CH_2O-$ | $C_6H_5$ | |
| 200a | $CH_3$ | 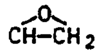 | $-OCH_2-$ | $C_6H_5$ | |
| 201a | 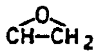 | $CH_3$ | $-OCH_2-$ | $C_6H_5$ | |
| 202a | $CH_3$ | 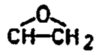 | $-CO-OCH_2-$ | $C_6H_5$ | |
| 203a | 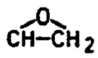 | $CH_3$ | $-CO-OCH_2-$ | $C_6H_5$ | |
| 204a | $CH_3$ | 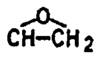 | $-CO-OCH_2-$ | 1-Methylcyclopropyl | |
| 205a | 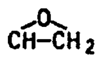 | $CH_3$ | $-CO-OCH_2-$ | 1-Methylcyclopropyl | |
| 206a | $SCH_3$ | $SCH_3$ | $-OCH_2-$ | $3-Cl-C_6H_4$ | öl; 1H-NMR($CDCl_3$): $\delta$=2.13(3H), 2.45(3H), 3.68(3H), 5.05(2H), 6.8-7.5(8H) |

Tabelle 3

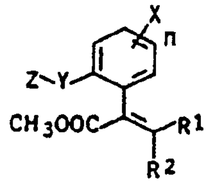

(X = Wasserstoff)

| Nr. | $R^1$ $R^2$ | Y | Z | Daten |
|---|---|---|---|---|
| 1b | $-(CH_2)_3-$ | -CH=CH- | $C_6H_5$ | |
| 2b | $-(CH_2)_3-$ | $-CH_2CH_2$ | $C_6H_5$ | |
| 3b | $-(CH_2)_3-$ | $-CH_2O-$ | $C_6H_5$ | |
| 4b | $-(CH_2)_3-$ | $-O-CH_2-$ | $C_6H_5$ | |
| 5b | $-(CH_2)_3-$ | $-CO-OCH_2-$ | $C_6H_5$ | |
| 6b | $-(CH_3)_3-$ | $-CO-OCH_2-$ | 1-Methylcyclopropyl | |
| 7b | $-(CH_2)_4-$ | -CH=CH- | $C_6H_5$ | |
| 8b | $-(CH_2)_4-$ | $-CH_2CH_2-$ | $C_6H_5$ | |
| 9b | $-(CH_2)_4-$ | $-CH_2O-$ | $C_6H_5$ | |
| 10b | $-(CH_2)_4-$ | $-OCH_2-$ | $C_6H_5$ | |
| 11b | $-(CH_2)_4-$ | $-CO-OCH_2-$ | $C_6H_5$ | |
| 12b | $-(CH_2)_4-$ | $-CO-OCH_2-$ | 1-Methylcyclopropyl | |
| 13b | $-(CH_2)_5-$ | -CH=CH- | $C_6H_5$ | |
| 14b | $-(CH_2)_5-$ | $-CH_2CH_2-$ | $C_6H_5$ | |
| 15b | $-(CH_2)_5-$ | $-CH_2O-$ | $C_6H_5$ | |
| 16b | $-(CH_2)_5-$ | $-OCH_2-$ | $C_6H_5$ | |
| 17b | $-(CH_2)_5-$ | $-CO-OCH_2-$ | $C_6H_5$ | |
| 18b | $-(CH_2)_5-$ | $-CO-OCH_2-$ | 1-Methylcyclopropyl | |
| 19b | -CH=CH-CH=CH- | -CH=CH- | $C_6H_5$ | |
| 20b | -CH=CH-CH=CH- | $-CH_2CH_2-$ | $C_6H_5$ | |
| 21b | -CH=CH-CH=CH- | $-CH_2O-$ | $C_6H_5$ | |
| 22b | -CH=CH-CH=CH- | $-OCH_2-$ | $C_6H_5$ | |
| 23b | -CH=CH-CH=CH- | $-CO-OCH_2-$ | $C_6H_5$ | |
| 24b | -CH=CH-CH=CH- | $-CO-OCH_2-$ | 1-Methylcyclopropyl | |
| 25b | $-CH_2CH_2OCH_2CH_2-$ | -CH=CH- | $C_6H_5$ | |
| 26b | $-CH_2CH_2OCH_2CH_2-$ | $-CH_2CH_2-$ | $C_6H_5$ | |
| 27b | $-CH_2CH_2OCH_2CH_2-$ | $-CH_2O-$ | $C_6H_5$ | |
| 28b | $-CH_2CH_2OCH_2CH_2-$ | $-OCH_2-$ | $C_6H_5$ | |
| 29b | $-CH_2CH_2OCH_2CH_2-$ | $-CO-OCH_2-$ | $C_6H_5$ | |
| 30b | $-CH_2CH_2OCH_2CH_2-$ | $-CO-OCH_2-$ | 1-Methylcyclopropyl | |
| 31b | $-OCH_2CH_2O-$ | -CH=CH- | $C_6H_5$ | |
| 32b | $-OCH_2CH_2O-$ | $-CH_2CH_2-$ | $C_6H_5$ | |
| 33b | $-OCH_2CH_2O-$ | $-CH_2O-$ | $C_6H_5$ | |
| 34b | $-OCH_2CH_2O-$ | $-OCH_2-$ | $C_6H_5$ | |
| 35b | $-OCH_2CH_2O-$ | $-CO-OCH_2-$ | $C_6H_5$ | |

| Nr. | R1 R2 | Y | Z | Daten |
|---|---|---|---|---|
| 36b | $-OCH_2CH_2O-$ | $-CO-OCH_2-$ | 1-Methylcyclopropyl | |
| 37b | $-O(CH_2)_3O-$ | $-CH=CH-$ | $C_6H_5$ | |
| 38b | $-O(CH_2)_3O-$ | $-CH_2CH_2-$ | $C_6H_5$ | |
| 39b | $-O(CH_2)_3O-$ | $-CH_2O-$ | $C_6H_5$ | |
| 40b | $-O(CH_2)_3O-$ | $-OCH_2-$ | $C_6H_5$ | |
| 41b | $-O(CH_2)_3O-$ | $-CO-OCH_2-$ | $C_6H_5$ | |
| 42b | $-O(CH_2)_3O-$ | $-CO-OCH_2-$ | 1-Methylcyclopropyl | |
| 43b | $-SCH_2CH_2S-$ | $-CH=CH-$ | $C_6H_5$ | |
| 44b | $-SCH_2CH_2S-$ | $-CH_2CH_2-$ | $C_6H_5$ | |
| 45b | $-SCH_2CH_2S-$ | $-CH_2O-$ | $C_6H_5$ | |
| 46b | $-SCH_2CH_2S-$ | $-OCH_2-$ | $C_6H_5$ | |
| 47b | $-SCH_2CH_2S-$ | $-CO-OCH_2$ | $C_6H_5$ | |
| 48b | $-SCH_2CH_2S-$ | $-CO-OCH_2-$ | 1-Methylcyclopropyl | |
| 49b | $-S(CH_2)_3S-$ | $-CH=CH-$ | $C_6H_5$ | |
| 50b | $-S(CH_2)_3S-$ | $-CH_2CH_2-$ | $C_6H_5$ | |
| 51b | $-S(CH_2)_3S-$ | $-CH_2O-$ | $C_6H_5$ | |
| 52b | $-S(CH_2)_3S-$ | $-OCH_2-$ | $C_6H_5$ | |
| 53b | $-S(CH_2)_3S-$ | $-CO-OCH_2-$ | $C_6H_5$ | |
| 54b | $-S(CH_2)_3S-$ | $-CO-OCH_2-$ | 1-Methylcyclopropyl | |
| 55b | $-CH_2OCH_2CH_2-$ | $-CH=CH-$ | $C_6H_5$ | |
| 56b | $-CH_2CH_2OCH_2-$ | $-CH=CH-$ | $C_6H_5$ | |
| 57b | $-CH_2OCH_2CH_2-$ | $-CH_2CH_2-$ | $C_6H_5$ | |
| 58b | $-CH_2CH_2OCH_2-$ | $-CH_2CH_2-$ | $C_6H_5$ | |
| 59b | $-CH_2OCH_2CH_2-$ | $-CH_2O-$ | $C_6H_5$ | |
| 60b | $-CH_2CH_2OCH_2-$ | $-CH_2O-$ | $C_6H_5$ | |
| 61b | $-CH_2OCH_2CH_2-$ | $-OCH_2-$ | $C_6H_5$ | |
| 62b | $-CH_2CH_2OCH_2-$ | $-OCH_2-$ | $C_6H_5$ | |
| 63b | $-CH_2OCH_2CH_2-$ | $-CO-OCH_2-$ | $C_6H_5$ | |
| 64b | $-CH_2CH_2OCH_2-$ | $-CO-OCH_2-$ | $C_6H_5$ | |
| 65b | $-CH_2OCH_2CH_2-$ | $-CO-OCH_2-$ | 1-Methylcyclopropyl | |
| 66b | $-CH_2CH_2OCH_2-$ | $-CO-OCH_2-$ | 1-Methylcyclopropyl | |
| 67b | $-COOCH_2CH_2-$ | $-CH=CH-$ | $C_6H_5$ | |
| 68b | $-CH_2CH_2OCO-$ | $-CH=CH-$ | $C_6H_5$ | |
| 69b | $-COOCH_2CH_2-$ | $-CH_2CH_2-$ | $C_6H_5$ | |
| 70b | $-CH_2CH_2OCO-$ | $-CH_2CH_2-$ | $C_6H_5$ | |
| 71b | $-COOCH_2CH_2-$ | $-CH_2O-$ | $C_6H_5$ | |
| 72b | $-CH_2CH_2OCO-$ | $-CH_2O-$ | $C_2H_5$ | |
| 73b | $-COOCH_2CH_2-$ | $-OCH_2-$ | $C_6H_5$ | |
| 74b | $-CH_2CH_2OCO-$ | $-OCH_2-$ | $C_6H_5$ | |
| 75b | $-COOCH_2CH_2-$ | $-CO-OCH_2-$ | $C_6H_5$ | |
| 76b | $-CH_2CH_2OCO-$ | $-CO-OCH_2-$ | $C_6H_5$ | |
| 77b | $-COOCH_2CH_2-$ | $-CO-OCH_2-$ | 1-Methylcyclopropyl | |
| 78b | $-CH_2CH_2OCO-$ | $-CO-OCH_2-$ | 1-Methylcyclopropyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender pflanzenkrankheiten :

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen pflanzen,
Plasmopara viticola an Reben,
Alternaria-Arten an Gemüse und Obst.

Die Verbindungen werden angewendet, indem man die pflanzen mit den Wirkstoffen besprüht oder bestäubt oder die Samen der pflanzen mit den Wirkstoffen behandelt. Die Anwendung erfolgt vor oder nach der Infektion der pflanzen oder Samen durch die pilze.

Die neuen Substanzen können in die üblichen Formulierungen übergeführt werden, wie Lösungen, Emulsionen, Suspensionen, Stäube, Pulver, pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z.B. xylol), chlorierte Aromaten (z.B. Chlorbenzole), paraffine (z.B. Erdölfraktionen), Alkohole (z.B. Methanol, Butanol), Ketone (z.B. Cyclohexanon), Amine (z.B. Ethanolamin, Dimethylformamid) und Wasser ; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, pulver, Stäube, pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gew.-Teile der Verbindung Nr. 151 (Tab. 1) mit 10 Gew.-Teilen N-Methyl-α-pyrrolidon und erhält eine Lösung, die zur Anwendung in Form kleinster Tropfen geeignet ist.

II. 20 Gew.-Teile der verbindung Nr. 151 (Tab. 1) werden in einer Mischung gelöst, die aus 80 Gew.-Teilen xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen

der Lösung in Wasser erhält man eine wäßrige Dispersion.

III. 20 Gew.-Teile der Verbindung Nr. 151 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 151 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 151 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 151 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 151 werden mit einer Mischung aus 92 Gew.-Teilen pulverförmigen Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 151 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 151 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbanat),
Ammoniak-Komplex von Zink-(N,N'-propylen-bis-dithiocarbamat),
Zink-(N,N'-propylen-bis-dithiocarbamat),
N,N'-polypropylen-bis-(thiocarbamoyl)-disulfid ;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,

2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl1-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde N-Tridecyl-2,6-dimethylmorpholin (A) – bekannt aus DE 1 164 152 – benutzt.

Anwendungsbeispiel

Wirksamkeit gegen Pyricularia oryzae (protektiv)

Blätter von in Töpfen gewachsenen Reiskeimlingen der Sorte "Bahia" wurden mit wäßrigen Emulsionen, die 80% Wirkstoff und 20% Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99% relativer Luftfeuchtigkeit aufgestellt. Nach 6 Tagen wurde das Ausmaß des Krankheitsbefalls ermittelt.

Das Ergebnis zeigt, daß der Wirkstoff 151 (Tab. 1) bei der Anwendung als 0,05 beige (Gew.%) Spritzbrühe eine bessere fungizide Wirkung zeigt (80%) als der bekannte vergleichswirkstoff A (40%).

**Ansprüche**

1. Acrylester der allgemeinen Formel I

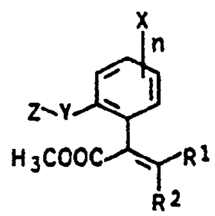

(I)

in der

$R^1$ und $R^2$ Halogen, $C_{1-4}$-Alkyl, $C_{1-3}$-Alkoxy, $C_{1-3}$-Alkylthio, Oxiranyl, gegebenenfalls substituiert durch Halogen, Hydroxy, $C_{1-3}$-Alkoxy, $C_{1-3}$-Halogenalkyl oder $C_{1-3}$-Halogenalkoxy bedeuten oder $R^1$ und $R^2$ zusammen einen gegebenenfalls ungesättigten 3- bis 6-gliedrigen Ring bedeuten, der gegebenenfalls 1 bis 3 Heteroatome O oder $S(O)_m$ (m = 0, 1 oder 2) enthält und der gegebenenfalls durch Halogen, Hydroxy, $C_{1-3}$-Alkyl, $C_{1-3}$-Alkoxy, $C_{1-3}$-Halogenalkyl oder Oxo substituiert ist, und

X Wasserstoff, Halogen, Cyano, Nitro, gegebenenfalls substituiertes $C_1$-$C_4$-Alkyl, gegebenenfalls substituiertes $C_1$-$C_4$-Alkoxy, gegebenenfalls substituiertes Halogen-$C_1$-$C_4$-alkyl,

n 1, 2, 3 oder 4,

y gegebenenfalls substituiertes $C_1$-$C_4$-Alkylen, $C_2$-$C_4$-Alkenylen, $C_2$-$C_4$-Alkinylen, O, $S(O)_p$ (p = 0, 1 oder 2), gegebenenfalls $C_1$-$C_4$-alkylsubstituiertes N, Oxytarbonyl, Carbonyloxy, Carbonyloxy-$C_1$-$C_4$-alkylen, Oxytarbonyl-$C_1$-$C_4$-alkylen, Oxy-$C_1$-$C_4$-alkylen, Thio-$C_1$-$C_4$-alkylen, Azo, Carbonylamino, Aminotarbonyl,

$>NCOO-$,

Methylenoxy, Methylenthio und

Z Wasserstoff, Alkyl, Cytloalkyl, Aryl, Arylalkyl, Aralkenyl, Aralkoxy, Aryloxy, Aryloxyalkyl, Hetaryl, Alkoxy, Alkoxyalkyl, Alkylthio, Halogenalkyl, gegebenenfalls substituiert durch Halogen, Cyano, Nitro, Alkyl, Alkenyl, Halogenalkenyl, Alkoxy, Halogenalkyl, Alkoxycarbonyl, gegebenenfalls substituiertes Phenyl bedeutet.

2. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge einer Verbindung der Formel I

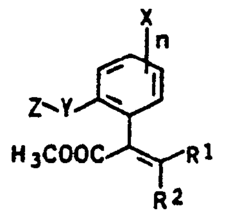

(I)

behandelt, in der

$R^1$ und $R^2$ Halogen, $C_{1-4}$-Alkyl, $C_{1-3}$-Alkoxy, $C_{1-3}$-Alkylthio, Oxiranyl gegebenenfalls substituiert durch Halogen, Hydroxy, $C_{1-3}$-Alkoxy, $C_{1-3}$-Halogenalkyl oder $C_{1-3}$-Halogenalkoxy bedeuten oder $R^1$ und $R^2$ zusammen einen gegebenenfalls ungesättigten 3- bis 6-gliedrigen Ring bedeuten, der gegebenenfalls 1 bis 3 Heteroatome O oder $S(O)_m$ (m = 0, 1 oder 2) enthält und der gegebenenfalls durch Halogen, Hydroxy, $C_{1-3}$-Alkyl, $C_{1-3}$-Alkoxy, $C_{1-3}$-Halogenalkyl oder Oxo substituiert ist, und

X Wasserstoff, Halogen, Cyano, Nitro, gegebenenfalls substituiertes $C_1$-$C_4$-Alkyl, gegebenenfalls substituiertes $C_1$-$C_4$-Alkoxy, gegebenenfalls substituiertes Halogen-$C_1$-$C_4$-alkyl,

n 1, 2, 3 oder 4,

Y gegebenenfalls substituiertes $C_1$-$C_4$-Alkylen, $C_2$-$C_4$-Alkenylen, $C_2$-$C_4$-Alkinylen, 0, $S(O)_p$ (p = 0, 1 oder 2), gegebenenfalls $C_1$-$C_4$-alkylsubstituiertes N, Oxycarbonyl, Carbonyloxy, Carbonyloxy-$C_1$-$C_4$-alkylen, Oxytarbonyl-$C_1$-$C_4$-alkylen. Oxy-$C_1$-$C_4$-alkylen, Thio-$C_1$-$C_4$-alkylen, Azo, Carbonylamino, Aminocarbonyl,

>NCOO-,

Methylenoxy, Methylenthio und

Z Wasserstoff, Alkyl, Cytloalkyl, Aryl, Arylalkyl, Aralkenyl, Aralkoxy, Aryloxy, Aryloxyalkyl, Hetaryl, Alkoxy, Alkoxyalkyl, Alkylthio, Halogenalkyl, gegebenenfalls substituiert durch Halogen, Cyano, Nitro, Alkyl, Alkenyl, Halogenalkenyl, Alkoxy, Halogenalkyl, Alkoxycarbonyl, gegebenenfalls substituiertes Phenyl bedeutet.

3. Fungizid, enthaltend einen inerten Trägerstoff und eine fungizid wirksame Menge einer Verbindung der Formel I

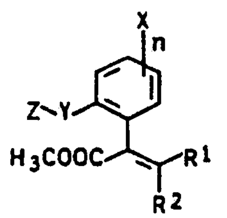

(I)

in der

$R^1$ und $R^2$ Halogen, $C_{1-4}$-Alkyl, $C_{1-3}$-Alkoxy, $C_{1-3}$-Alkylthio, Oxiranyl gegebenenfalls substituiert durch Halogen, Hydroxy, $C_{1-3}$-Alkoxy, $C_{1-3}$-Halogenalkyl oder $C_{1-3}$-Halogenalkoxy bedeuten oder $R^1$ und $R^2$ zusammen einen gegebenenfalls ungesättigten 3- bis 6-gliedrigen Ring bedeuten, der gegebenenfalls 1 bis 3 Heteroatome O oder $S(O)_m$ (m = 0, 1 oder 2) enthält und der gegebenenfalls durch Halogen, Hydroxy, $C_{1-3}$-Alkyl, $C_{1-3}$-Alkoxy, $C_{1-3}$-Halogenalkyl oder Oxo substituiert ist, und

X Wasserstoff, Halogen, Cyano, Nitro, gegebenenfalls substituiertes $C_1$-$C_4$-Alkyl, gegebenenfalls substituiertes $C_1$-$C_4$-Alkoxy, gegebenenfalls substituiertes Halogen-$C_1$-$C_4$-alkyl,

n 1, 2, 3 oder 4,

Y gegebenenfalls substituiertes $C_1$-$C_4$-Alkylen, $C_2$-$C_4$-Alkenylen, $C_2$-$C_4$-Alkinylen, O, $S(O)_p$ (p = 0, 1 oder 2), gegebenenfalls $C_1$-$C_4$-alkylsubstituiertes N, Oxycarbonyl, Carbonyloxy, Carbonyloxy-$C_1$-$C_4$-alkylen, Oxytarbonyl-$C_1$-$C_4$-alkylen, Oxy-$C_1$-$C_4$-alkylen,Thio-$C_1$-$C_4$-alkylen, Azo, Carbonylamino, Aminotarbonyl,

>NCOO-,

Methylenoxy, Methylenthio und

Z Wasserstoff, Alkyl, Cytloalkyl, Aryl, Arylalkyl, Aralkenyl, Aralkoxy, Aryloxy, Aryloxyalkyl, Hetaryl, Alkoxy,

Alkoxyalkyl, Alkylthio, Halogenalkyl, gegebenenfalls substituiert durch Halogen, Cyano, Nitro, Alkyl, Alkenyl, Halogenalkenyl, Alkoxy, Halogenalkyl, Alkoxytarbonyl, gegebenenfalls substituiertes Phenyl bedeutet.

4. Verfahren zur Herstellung eines fungiziden Mittels, dadurch gekennzeichnet, daß man eine oder mehrere Verbindungen der Formel I gemäß Anspruch 1 mit einem festen oder flüssigen Trägerstoff mischt.

5. Verbindung der Formel I gemäß Anspruch 1, in der $R^1$ Methyl, $R^2$ Methyl, Y den Rest $-COOCH_2-$ und Z 1-Methyltyclopropyl bedeuten.

6. Verbindung der Formel I gemäß Anspruch 1, in der $R^1$ Methyl, $R^2$ Methyl, Y den Rest $-CH_2O-$ und Z $2-CH_3-C_6H_4$ bedeuten.

7. Verbindung der Formel I gemäß Anspruch 1, in der $R^1$ Methyl, $R^2$ Methyl, Y den Rest $-COOCH_2-$ und Z Phenyl bedeuten.

**Revendications**

1. Esters acryliques de la formule générale I

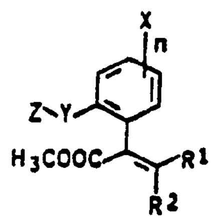

(I)

dans laquelle

$R^1$ et $R^2$ représentent halogène, alkyle en $C_{1-4}$, alcoxy en $C_{1-3}$, alkylthio en $C_{1-3}$, oxiranyle, éventuellement substitué par halogène, hydroxy, alcoxy en $C_{1-3}$, halogénalkyle en $C_{1-3}$ ou halogénalcoxy en $C_{1-3}$, ou $R^1$ et $R^2$ représentent ensemble un noyau à 3 à 6 chaînons, éventuellement insaturé, pouvant contenir 1 à 3 hétéroatomes O ou $S(O)_m$ (m = 0, 1 ou 2) et qui est éventuellement substitué par halogène, hydroxy, alkyle en $C_{1-3}$, alcoxy en $C_{1-3}$, halogénalkyle en $C_{1-3}$ ou oxo, et

X représente hydrogène, halogène, cyano, nitro, alkyle en $C_{1-4}$ éventuellement substitué, alcoxy en $C_{1-4}$ éventuellement substitué, halogénalkyle en $C_{1-4}$ éventuellement substitué,

n = 1 à 4,

Y représente alkylène en $C_{1-4}$ éventuellement substitué, alcénylène en $C_{2-4}$, alcynylène en $C_{2-4}$, O, $S(O)_p$ (p = 0, 1 ou 2), N éventuellement substitué par alkyle en $C_{1-4}$, oxycarbonyle, carbonyloxy, carbonyloxy-alkylène en $C_{1-4}$, oxycarbonyl-alkylène en $C_{1-4}$, oxy-alkylène en $C_{1-4}$, thio-alkylène en $C_{1-4}$, azo, carbonylamino, aminocarbonyle,

$$>NCOO-,$$

méthylènoxy, méthylènethio et

Z représente hydrogène, alkyle, cycloalkyle, aryle, arylalkyle, aralcényle, aralcoxy, aryloxy, aryloxyalkyle, hétaryle, alcoxy, alcoxyalkyle, alkylthio, halogénalkyle, ces restes étant éventuellement substitués par halogène, cyano, nitro, alkyle, alcényle, halogénalcényle, alcoxy, halogénalkyle, phényle éventuellement substitué, alcoxycarbonyle.

2. Procédé de lutte contre les champignons, caractérisé par le fait que l'on traite les champignons ou les matériaux, plantes, semences ou le sol avec une quantité efficace du point de vue fongicide d'un composé de formule I

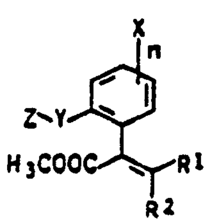

(I)

dans laquelle

$R^1$ et $R^2$ représentent halogène, alkyle en $C_{1-4}$, alcoxy en $C_{1-3}$, alkylthio en $C_{1-3}$, oxiranyle, éventuellement substitué par halogène, hydroxy, alcoxy en $C_{1-3}$, halogénalkyle en $C_{1-3}$ ou halogénalcoxy en $C_{1-3}$, ou $R^1$ et $R^2$ représentent ensemble un noyau à 3 à 6 chaînons, éventuellement insaturé, pouvant contenir 1 à 3 hétéroatomes O ou $S(O)_m$ (m = 0, 1 ou 2) et qui est éventuellement substitué par halogène, hydroxy, alkyle en $C_{1-3}$, alcoxy en $C_{1-3}$, halogénalkyle en $C_{1-3}$ ou oxo, et

X représente hydrogène, halogène, cyano, nitro, alkyle en $C_{1-4}$ éventuellement substitué, alcoxy en $C_{1-4}$ éventuellement substitué, halogénalkyle en $C_{1-4}$ éventuellement substitué,

n = 1 à 4,

y représente alkylène en $C_{1-4}$ éventuellement substitué, alcénylène en $C_{2-4}$, alcynylène en $C_{2-4}$, O, $S(O)_p$ (p = 0, 1 ou 2), N éventuellement substitué par alkyle en $C_{1-4}$, oxycarbonyle, carbonyloxy, carbonyloxy-alkylène en $C_{1-4}$, oxycarbonyl-alkylène en $C_{1-4}$, oxy-alkylène en $C_{1-4}$, thio-alkylène en $C_{1-4}$, azo, carbonylamino, aminocarbonyle,

$$>NCOO-,$$

méthylènoxy, méthylènethio et

Z représente hydrogène, alkyle, cycloalkyle, aryle, arylalkyle, aralcényle, aralcoxy, aryloxy, aryloxyalkyle, hétaryle, alcoxy, alcoxyalkyle, alkylthio, halogénalkyle, ces restes étant éventuellement substitués par halogène, cyano, nitro, alkyle, alcényle, halogénalcényle, alcoxy, halogénalkyle, phényle éventuellement substitué, alcoxycarbonyle.

3. Fongicide, contenant un véhicule inerte et une quantité efficace du point de vue fongicide d'un composé de formule I

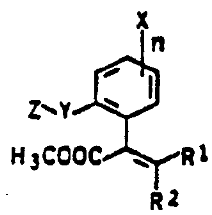

(I)

dans laquelle

$R^1$ et $R^2$ représentent halogène, alkyle en $C_{1-4}$, alcoxy en $C_{1-3}$, alkylthio en $C_{1-3}$, oxiranyle, éventuellement substitué par halogène, hydroxy, alcoxy en $C_{1-3}$, halogénalkyle en $C_{1-3}$ ou halogénalcoxy en $C_{1-3}$, ou $R^1$ et $R^2$ représentent ensemble un noyau à 3 à 6 chaînons, éventuellement insaturé, pouvant contenir 1 à 3 hétéroatomes O ou $S(O)_m$ (m = 0, 1 ou 2) et qui est éventuellement substitué par halogène, hydroxy, alkyle en $C_{1-3}$, alcoxy en $C_{1-3}$, halogénalkyle en $C_{1-3}$ ou oxo, et

X représente hydrogène, halogène, cyano, nitro, alkyle en $C_{1-4}$ éventuellement substitué, alcoxy en $C_{1-4}$ éventuellement substitué, halogénalkyle en $C_{1-4}$ éventuellement substitué,

n = 1 à 4,

Y représente alkylène en $C_{1-4}$ éventuellement substitué, alcénylène en $C_{2-4}$, alcynylène en $C_{2-4}$, O, $S(O)_p$ (p = 0, 1 ou 2), N éventuellement substitué par alkyle en $C_{1-4}$, oxycarbonyle, carbonyloxy, carbonyloxy-alkylène en $C_{1-4}$, oxycarbonyl-alkylène en $C_{1-4}$, oxy-alkylène en $C_{1-4}$, thio-alkylène en $C_{1-4}$, azo, carbonylamino, aminocarbonyle,

$$>NCOO-,$$

méthylènoxy, méthylènethio et

Z représente hydrogène, alkyle, cycloalkyle, aryle, arylalkyle, aralcényle, aralcoxy, aryloxy, aryloxyalkyle, hétaryle, alcoxy, alcoxyalkyle, alkylthio, halogénalkyle, ces restes étant éventuellement substitués par halogène, cyano, nitro, alkyle, alcényle, halogénalcényle, alcoxy, halogénalkyle, phényle éventuellement substitué, alcoxycarbonyle.

4. Procédé de préparation d'un fongicide, caractérisé par le fait que l'on mélange un ou plusieurs composés de formule I, selon la revendication 1, avec un véhicule solide ou liquide.

5. Composé de formule I, selon la revendication 1, dans lequel $R^1$ représente méthyle, $R^2$, méthyle, y, le

reste $-COOCH_2$ et Z, 1-méthylcyclopropyle.

6. Composé de formule I, selon la revendication 1, dans lequel $R^1$ représente méthyle, $R^2$, méthyle, y, le reste $-CH_2O-$ et Z, $2\text{-}CH_3\text{-}C_6H_4$.

7. Composé de formule I, selon la revendication 1, dans lequel $R^1$ représente méthyle, $R^2$, méthyle, y, le reste $-COOCH_2-$ et Z, phényle.

## Claims

1. Acrylates of the formula I

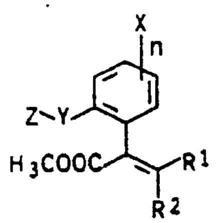

(I)

where

$R^1$ and $R^2$ are halogen, or $C_{1-4}$-alkyl, $C_{1-3}$-alkoxy, $C_{1-3}$-alkylthio or oxiranyl, unsubstituted or substituted by halogen, hydroxy, $C_{1-3}$-alkoxy, $C_{1-3}$-haloalkyl or $C_{1-3}$-haloalkoxy, or $R^1$ and $R^2$ together denote a saturated or unsaturated 3 to 6 -membered ring which may contain from 1 to 3 heteroatoms O or $S(O)_m$ ($m$ = 0, 1 or 2) and which is unsubstituted or substituted by halogen, hydroxy, $C_{1-3}$-alkyl, $C_{1-3}$-alkoxy, $C_{1-3}$-haloalkyl or oxo, and

X is hydrogen, halogen, cyano, nitro, substituted or unsubstituted $C_1$-$C_4$4 alkyl, substituted or unsubstituted $C_1$-$C_4$-alkoxy, substituted or unsubstituted halo-$C_1$-$C_4$-alkyl,

n is 1, 2, 3 or 4,

Y is substituted or unsubstituted $C_1$-$C_4$-alkylene,$C_2$-$C_4$-alkenylene, $C_2$-$C_4$-alkynylene, O, $S(O)_p$ ($p$ = 0, 1 or 2) unsubstituted or $C_1$-$C_4$-alkyl-substituted N, oxycarbonyl, carbonyloxy, carbonyloxy-$C_1$-$C_4$-alkylene, oxycarbonyl-$C_1$-$C_4$-alkylene, oxy-$C_1$-$C_4$-alkylene, thio-$C_1$-$C_4$-alkylene, azo, carbonylamino, aminocarbonyl,

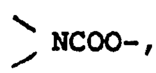

methyleneoxy or methylenethio and

Z is hydrogen, alkyl, cycloalkyl, aryl, aralkyl, aralkenyl, aralkoxy, aryloxy, aryloxyalkyl, hetaryl, alkoxy, alkoxyalkyl, alkylthio, or haloalkyl, these radicals being unsubstituted or substituted by halogen, cyano, nitro, alkyl, alkenyl, haloalkenyl, alkoxy, haloalkyl, alkoxycarbonyl or unsubstituted or substituted phenyl.

2. A process for combating fungi, wherein the fungi, or the materials, plants, seed or the soil at risk from fungus attack are treated with a fungicidally effective amount of a compound of the formula I

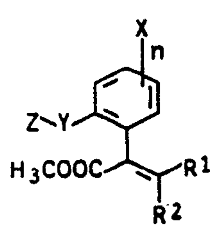

(I)

where

$R^1$ and $R^2$ are halogen, $C_{1-4}$-alkyl, $C_{1-3}$-alkoxy, $C_{1-3}$-alkylthio or oxiranyl, unsubstituted or substituted by halogen, hydroxy, $C_{1-3}$-alkoxy, $C_{1-3}$-haloalkyl or $C_{1-3}$-haloalkoxy, or $R^1$ and $R^2$ together denote a saturated or unsaturated 3-to 6- membered ring which may contain from 1 to 3 heteroatoms O or $S(O)_m$ ($m$ = 0, 1, 2) and which is unsubstituted or substituted by halogen, hydroxy, $C_{1-3}$-alkyl, $C_{1-3}$-alkoxy, $C_{1-3}$-haloalkyl or oxo, and

X is hydrogen, halogen, cyano, nitro, substituted or unsubstituted $C_1$-$C_4$-alkyl, substituted or unsubstituted

$C_1$-$C_4$-alkoxy, substituted or unsubstituted halo-$C_1$-$C_4$-alkyl,

n is 1, 2, 3 or 4,

Y is substituted or unsubstituted $C_1$-$C_4$-alkylene, $C_2$-$C_4$-alkenylene, $C_2$-$C_4$-alkynylene, O, $S(O)_p$ (p = 0, 1 or 2), unsubstituted or $C_1$-$C_4$-alkyl-substituted N, oxycarbonyl, carbonyloxy, carbonyloxy-$C_1$-$C_4$-alkylene, oxycarbonyl1-$C_1$-$C_4$-alkylene, oxy-$C_1$-$C_4$-alkylene, thio-$C_1$-$C_4$-alkylene, azo, carbonylamino, aminocarbonyl,

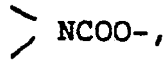

methyleneoxy or methylenethio and

Z is hydrogen, alkyl, cycloalkyl, aryl, aralkyl, aralkenyl, aralkoxy, aryloxy, aryloxyalkyl, hetaryl, alkoxy, alkoxyalkyl, alkylthio, or haloalkyl, these radicals being unsubstituted or substituted by halogen, cyano, nitro, alkyl, alkenyl, haloalkenyl, alkoxy, haloalkyl, alkoxycarbonyl or unsubstituted or substituted phenyl.

3. A fungicide containing an inert carrier and a fungicidally effective amount of a compound of the formula I

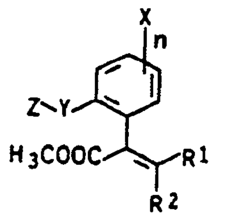

(I)

where

$R^1$ and $R^2$ are halogen, or $C_{1-4}$-alkyl, $C_{1-3}$-alkoxy, $C_{1-3}$-alkylthio or oxiranyl unsubstituted or substituted by halogen, hydroxy, $C_{1-3}$-alkoxy, $C_{1-3}$-haloalkyl or $C_{1-3}$-haloalkoxy, or $R^1$ and $R^2$ together denote a saturated or unsaturated 3- to 6-membered ring which may contain from 1 to 3 heteroatoms O or $S(O)_m$ (m = 0, 1 or 2) and which is unsubstituted or substituted by halogen, hydroxy, $C_{1-3}$-alkyl, $C_{1-3}$-alkoxy, $C_{1-3}$-haloalkyl or oxo, and

X is hydrogen, halogen, cyano, nitro, substituted or unsubstituted $C_1$-$C_4$-alkyl, substituted or unsubstituted $C_1$-$C_4$-alkoxy, substituted or unsubstituted halo-$C_1$-$C_4$-alkyl,

n is 1, 2, 3 or 4,

Y is substituted or unsubstituted $C_1$-$C_4$-alkylene, $C_2$-$C_4$-alkenylene, $C_2$-$C_4$-alkynylene, O, $S(O)_p$ (p = 0, 1 or 2), unsubstituted or $C_1$-$C_4$-alkyl-substituted N, oxycarbonyl, carbonyloxy, carbonyloxy-$C_1$-$C_4$-alkylene, oxycarbonyl-$C_1$-$C_4$-alkylene, oxy-$C_1$-$C_4$-alkylene, thio-$C_1$-$C_4$-alkylene, azo, carbonylamino, aminocarbonyl,

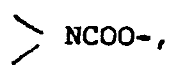

methyleneoxy or methylenethio and

Z is hydrogen, alkyl, cycloalkyl, aryl, aralkyl, aralkenyl, aralkoxy, aryloxy, aryloxyalkyl, hetaryl, alkoxy, alkoxyalkyl, alkylthio, or haloalkyl, these radicals being unsubstituted or substituted by halogen, cyano, nitro, alkyl, alkenyl, haloalkenyl, alkoxy, haloalkyl, alkoxycarbonyl or unsubstituted or substituted phenyl.

4. A process for the preparation of a fungicide, which comprises mixing one or more compounds of the formula I as claimed in claim 1 with a solid or liquid carrier.

5. A compound of the formula I as claimed in claim 1, where $R^1$ is methyl, $R^2$ is methyl, Y is $-COOCH_2-$ and Z is 1-methylcyclopropyl.

6. A compound of the formula I as claimed in claim 1, where $R^1$ is methyl, $R^2$ is methyl, Y is $-CH_2O-$ and Z is 2-$CH_3$-$C_6H_4$.

7. A compound of the formula I as claimed in claim 1, where $R^1$ is methyl, $R^2$ is methyl, Y is $-COOCH_2-$ and Z is phenyl.